# EUROPEAN PATENT SPECIFICATION

(11) **EP 3 993 694 B1**
(45) Date of publication and mention of the grant of the patent: **03.12.2025**
(21) Application number: 20834430.9
(22) Date of filing: 30.06.2020
(51) Int. Cl.: A61B 5/02, A61B 5/00, A61M 1/02, G06T 7/00

(54) **PROVIDING GUIDANCE DURING A MEDICAL PROCEDURE**
BEREITSTELLUNG EINER ANLEITUNG WÄHREND EINES MEDIZINISCHEN VERFAHRENS
FOURNITURE DE GUIDAGE PENDANT UNE PROCÉDURE MÉDICALE

(30) Priority: 03.07.2019 US 201962870608 P
(43) Date of publication of application: 11.05.2022
(73) Proprietor: Stryker Corporation, Portage, Michigan 49002 (US)
(72) Inventor: SATISH, Siddarth, Redwood City, California 94061 (US); HOSFORD, Andrew T., Idaho Falls, Idaho 83406 (US); CARROLL, Charles Peterson, Berkeley, California 94709 (US); HYOUNG, Peter S., Oakland, California 94619 (US); KROPILAK, John G., Cary, North Carolina 27518 (US)
(74) Representative: Röthinger, Rainer
(86) International application number: PCT/US2020/040248
(87) International publication number: WO 2021/003130

(56) References cited:
- US-A1- 2013 010 094
- US-A1- 2015 154 751
- US-A1- 2015 221 088
- US-A1- 2016 331 248
- US-A1- 2016 331 248
- US-A1- 2017 185 739
- US-A1- 2017 185 739
- US-A9- 2017 367 593
- US-B2- 8 792 693

## Description

### TECHNICAL FIELD

The subject matter disclosed herein generally relates to the technical field of medical care, and more specifically to new and useful systems and methods for providing guidance during a medical procedure. Such systems include special-purpose machines that facilitate providing guidance during a medical procedure, including software-configured computerized variants of such special-purpose machines and improvements to such variants, and to the technologies by which such special-purpose machines become improved compared to other special-purpose machines that facilitate providing guidance during a medical procedure.

### BACKGROUND

Effectively managing and responding to patient blood loss during medical procedures helps avoid complications. However, effective management and response to patient blood loss involves various challenges, such as inaccurate reporting of patient blood loss and misapplication of best practice procedures for blood loss management. For example, doctors and other medical personnel often visually estimate patient blood loss. This leads to significant error, which can result in a mischaracterization of the severity of a patient's hemorrhage risk and consequent failure to perform steps to effectively manage the patient's blood loss and prevent resulting complications. For example, underestimation of patient blood loss may lead to delayed resuscitation and transfusion, increased risk of infections, tissue death, or even patient death, such as in the event of hemorrhage. Thus, it may be beneficial to provide new and improved systems and methods for providing guidance during a medical procedure for improving patient outcomes.

US 2017/0185739 A1 describes a computerized method for postpartum hemorrhage risk assessment, the method comprising: referencing stored patient information to identify one or more risk factors associated with an increase in likelihood of postpartum hemorrhage occurrence; receiving an indication from a clinician-user to measure one or more items via a scale device; measuring a weight of the one or more items; determining a quantitative blood loss of a patient based on the weight of the one or more items and a predetermined dry weight of the one or more items; identifying a postpartum hemorrhage stage that corresponds to the quantitative blood loss of the patient; communicating the postpartum hemorrhage stage and the quantitative blood loss of the patient to the clinician user, and communicating one or more recommendations to the clinician-user, wherein the one or more recommendations are specific to the postpartum hemorrhage stage.

A method for communicating medical parameter information of a patient to a user is known from US 2017/0367593 A9. The method comprises: at a computing system, receiving data representative of a medical parameter; determining a statistical component associated with the medical parameter; at the computing system, updating an analysis of an aggregate amount of the medical parameter and an aggregate statistical component associated with the medical parameter, based upon the medical parameter and the statistical component; and, at a user interface device in communication with the computing system, providing information derived from the analysis of the aggregate amount of the medical parameter and the aggregate statistical component, to the user.

US 2016/0331248 A1 discloses a method of patient monitoring, comprising: displaying an estimate of real-time blood loss value for a patient corresponding to a first time point of a current surgical procedure; displaying an updated estimate of real-time blood loss value for the patient corresponding to a second time point after the first time point; and displaying at least one reference blood loss value corresponding to the first time point, the second time point, or a third time point after the second time point.

### SUMMARY

A method for providing guidance during a medical procedure is defined in claim 1 and a system for providing guidance during a medical procedure is defined in claim 7, whereas a machine-readable medium comprising instructions that, when executed by one or more processors of a machine, cause the machine to carry out the steps of the method of claim 1 is defined in claim 13. Optional features are defined in the dependent claims.

### BRIEF DESCRIPTION OF THE DRAWINGS

FIG. 1 is a schematic block diagram depicting a method for guiding a user (e.g., a doctor, such as a surgeon, or other medical personnel) during a medical procedure, according to some example embodiments.
FIG. 2 is a schematic depiction of a blood loss protocol, according to some example embodiments.
FIG. 3 is a schematic block diagram depicting a method for estimating a quantity of a blood component within a substrate, according to some example embodiments.
FIG. 4 is a schematic depiction of a blood loss protocol and methods of selecting elements from the blood loss protocol to provide guidance to a user, according to some example embodiments.
FIG. 5 is a schematic block diagram depicting a method for guiding a user during a medical procedure, according to some example embodiments.
FIG. 6 is a depiction of a graphical user interface (GUI) that includes elements of a blood loss protocol to guide a user, according to some example embodiments.
FIG. 7 is a depiction of a GUI that includes elements from multiple patient stages of a blood loss protocol to guide a user, according to some example embodiments.
FIG. 8 is a depiction of a summary graphic that may be displayed (e.g., by a GUI) to users during a medical procedure, according to some example embodiments.
FIG. 9 is a schematic depiction of a data record for storing and organizing data generated during a medical procedure, according to some example embodiments.
FIG. 10 is a schematic depiction of a system for guiding a user during a medical procedure, according to some example embodiments.
FIG. 11 is a schematic depiction of a blood loss protocol, according to some example embodiments.
FIG. 12 is a depiction of a GUI that includes elements of a blood loss protocol to guide a user, according to some example embodiments.
FIG. 13 is a depiction of a GUI that includes a summary graphic that may be displayed to users during a medical procedure, according to some example embodiments.
FIG. 14 is a depiction of a GUI allowing a user to manually input a fluid volume measured using a fluid container, according to some example embodiments.
FIG. 15 is a schematic block diagram depicting a method for guiding a user during a medical procedure, according to some example embodiments.

### DETAILED DESCRIPTION

Example methods (e.g., algorithms) facilitate providing guidance during a medical procedure (e.g., during a surgical procedure), and example systems (e.g., special-purpose machines configured by special-purpose software) are configured to facilitate providing guidance during a medical procedure. Examples merely typify possible variations. Unless explicitly stated otherwise, structures (e.g., structural components, such as modules) are optional and may be combined or subdivided, and operations (e.g., in a procedure, algorithm, or other function) may vary in sequence or be combined or subdivided. In the following description, for purposes of explanation, numerous specific details are set forth to provide a thorough understanding of various example embodiments. It will be evident to one skilled in the art, however, that the present subject matter may be practiced without these specific details.

Any one or more of the example methods and systems discussed herein may be used to provide guidance (e.g., to one or more users, such as one or more doctors or other medical personnel) during a medical procedure (e.g., a surgical procedure performed on a patient). One or more of the example methods may utilize one or more processors to carry out the operations (e.g., steps) specified for providing guidance during a medical procedure. Generally, example methods discussed herein may include estimating a blood loss of a patient (e.g., estimating an amount of blood lost by the patient), determining a current patient stage of a blood loss protocol (e.g., determining a current stage of the patient within a protocol for addressing blood loss) based on the estimated blood loss, and providing guidance to a user (e.g., a doctor) based on the current patient stage of the blood loss protocol. Any suitable blood loss protocol may be used to guide the user during the medical procedure. In some example embodiments, the blood loss protocol may be or include a predetermined protocol for managing hemorrhage risk (e.g., for the patient undergoing the medical procedure, for patients generally, or both). The blood loss protocol (e.g., for managing hemorrhage risk) may include a plurality of patient stages, which may be delineated based on the severity of the patient's blood loss. The blood loss protocol may be stored in one or more memory devices. In some example embodiments, the blood loss protocol may be accessible by one or more processors to provide guidance to the user. Further, the blood loss protocol may be customizable based on one or more user settings. For example, such user settings may include one or more threshold blood loss values that are associated with each of the plurality of patient stages of the blood loss protocol. Each of the plurality of patient stages of a blood loss protocol may be associated with a corresponding lower threshold blood loss value and a corresponding upper threshold blood loss value (e.g., to define a range of blood loss). Thus, the determining of the current patient stage may comprise comparing the estimated blood loss to the threshold values associated with one or more patient stages of the blood loss protocol.

In some example embodiments, the estimating of the patient blood loss includes estimating the amount of blood in a fluid-containing substrate, such as a fluid container or a surgical item such as a textile. Estimating the amount of fluid in the substrate may include the use of one or more image analysis techniques. For example, the estimating of the patient blood loss may include evaluating a color parameter in one or more images that depict the fluid- containing substrate. In some example embodiments, the estimating of the patient blood loss includes correlating a color parameter to a blood component concentration in the fluid-containing substrate to estimate the amount of blood contained in the substrate. For example, the estimating of the patient blood loss may include comparing a color parameter of at least a portion of an image to a template image to determine a blood component concentration associated with at least a portion of the fluid-containing substrate. As another example, the estimating of the amount of blood contained in the fluid-containing substrate may include using a parametric equation to determine a blood component concentration based on a color parameter. In some example embodiments, the estimating of the patient blood loss includes combining the amount of blood in a plurality of fluid-containing substrates. In some example embodiments, the determining of the patient blood loss includes detecting or determining a weight of the fluid-containing substrate. Weight information may be used instead of, or in conjunction with, information obtained from analyzing an image depicting the fluid-containing substrate. For example, one or more image analysis techniques may be used to determine a blood component concentration, and weight information may be used to determine a volume of a fluid in the fluid-containing substrate. The concentration and the volume may be used to determine the blood loss of the patient.

In some example embodiments of the methods and systems described herein for providing guidance to a user during a medical procedure, the estimated blood loss of the patient informs the guidance provided to the user, and the guidance may be based on a blood loss protocol. The blood loss protocol used to provide guidance to the user may include a plurality of patient stages. Each patient stage may comprise one or more elements. One or more of the elements in any given stage may be or include a recommended action (e.g., start a blood transfusion, administer an intravenous (IV) infusion, provide a particular medication to the patient, or any suitable combination thereof). Thus, in some example embodiments, the providing of the guidance includes selecting one or more elements from the current patient stage of the blood loss protocol. The guidance may be selected, generated, provided, or any suitable combination thereof, based on the estimated blood loss of the patient (e.g., by selecting elements from the current patient stage, which may be determined based on the estimated blood loss of the patient). However, in some example embodiments, the guidance may also be based on user input (e.g., one or more user inputs from one or more users). For example, a user input (e.g., an indication of an action that the user has taken, or patient information manually input by the user) may influence one or more of the elements selected by the processor to provide guidance to the user. The providing of the guidance to the user may further involve displaying the one or more elements selected from the current patient stage, or a previous or subsequent patient stage, of the protocol on a display (e.g., a display screen of a mobile computing device such as a laptop, a tablet, or a smartphone). Additionally or alternatively, the guidance may be provided in the example form of, or alongside with, other visual notifications (e.g., lights such as LEDs on a device), audio notifications (e.g., through one or more speakers), tactile notifications (e.g., vibrations, etc.), or any suitable combination thereof.

In some example embodiments of the methods and systems described herein, guiding the user through the blood loss protocol includes providing an updated guidance (e.g., based on an updated estimate of patient blood loss). The guidance to be provided may be updated in any suitable manner. For example, providing the guidance may include estimating an updated patient blood loss (e.g., updating an estimated blood loss for the patient), and determining an updated patient stage based on the updated estimated patient blood loss. Thus, the updated guidance may be provided based on the updated patient stage. In some example embodiments, the updated guidance may be provided based on one or more user inputs. For example, the updated guidance may be provided based on an indication that the user has performed one or more elements of the blood loss protocol. In some example embodiments, the updated guidance may be based on both a user input and an updated patient stage of the blood loss protocol. Providing the updated guidance may include displaying the updated guidance, such as on a display screen (e.g., a display screen of a mobile computing device such as a laptop, tablet, smartphone, or any suitable combination thereof). In some example embodiments, the providing of the guidance includes providing a notification associated with a current patient stage of the blood loss protocol. The guidance may be provided as a visual notification (e.g., on a display, one or more lights, or any suitable combination thereof), an audio notification (e.g., one or more audio alerts through one or more speakers), a tactile notification (e.g., one or more vibrations). For example, it may be desirable to periodically or intermittently inform the user of the current patient stage, when the current patient stage has changed, or both.

Also described herein are example systems for providing guidance to a user during a medical procedure. Generally, example systems discussed herein include one or more processors configured to estimate a blood loss of a patient, determine a current patient stage of a blood loss protocol based on the estimated blood loss, and provide guidance to a user based on the current patient stage of the blood loss protocol. The blood loss protocol may include a plurality of patient stages, and each patient stage may include one or more elements. For example, an element of a patient stage may be or include recommending one or more user actions. The providing of guidance to the user may include selecting (e.g., via a processor) one or more elements from the current patient stage of the blood loss protocol. Thus, one or more processors of one or more systems for providing guidance to the user may be configured to select one or more elements from the current patient stage of the blood loss protocol. Further, one or more processors may be configured to provide an updated guidance to the user based on a user input, an updated patient stage of the blood loss protocol, or both. In some example embodiments, the one or processors are configured to estimate an updated blood loss of the patient, and the one or more processors may determine the updated patient stage based on the updated patient blood loss. The example systems discussed herein may include a display configured to display information, such as the guidance (e.g., one or more elements from the current patient stage of the blood loss protocol) provided to the user.

Furthermore, in some example embodiments, the example methods discussed herein include estimating a medical parameter of the patient, determining a current patient stage of a medical parameter protocol based on the estimated medical parameter, and providing guidance to the user based on the current patient stage of the medical parameter protocol. Any suitable medical parameter protocol may be used to guide the user during the medical procedure. Similarly, in some example embodiments, the example systems discussed herein include one or more processors configured to estimate a medical parameter of the patient, determine a current patient stage of a medical parameter protocol based on the estimated medical parameter, and provide guidance to the user based on the current patient stage of the medical parameter protocol. Examples of such a medical parameter include blood loss, blood pressure, hematocrit, hemoglobin concentration, concentration of another blood component, heart rate, blood coagulation (e.g., characterized by thromboelastography (TEG)), temperature, oxygen saturation, height, weight, age, body mass index (BMI), sex, body fat percentage, intravenous fluid delivered, or any suitable combination thereof. One or more of such medical parameters may be estimated using one or more of the example methods described herein (e.g., estimated blood loss or estimated blood component concentration), using suitable instrumentation (e.g., sphygmomanometer, scale, pulse oximeter, thermometer, flow meter coupled to an IV setup, etc.), or any suitable combination thereof.

Any one or more of the example methods and systems discussed herein may be used to provide guidance to one or more users (e.g., one or more doctors or other medical personnel) during a medical procedure (e.g., performed on patient). Such methods and systems may utilize one or more processors to perform various operations (e.g., steps), such as estimating patient blood loss, determining a current patient stage of a blood loss protocol, and providing guidance to a user based on one or more of the current patient stage (e.g., further based on one or more user inputs).

### Overview of Example Methods

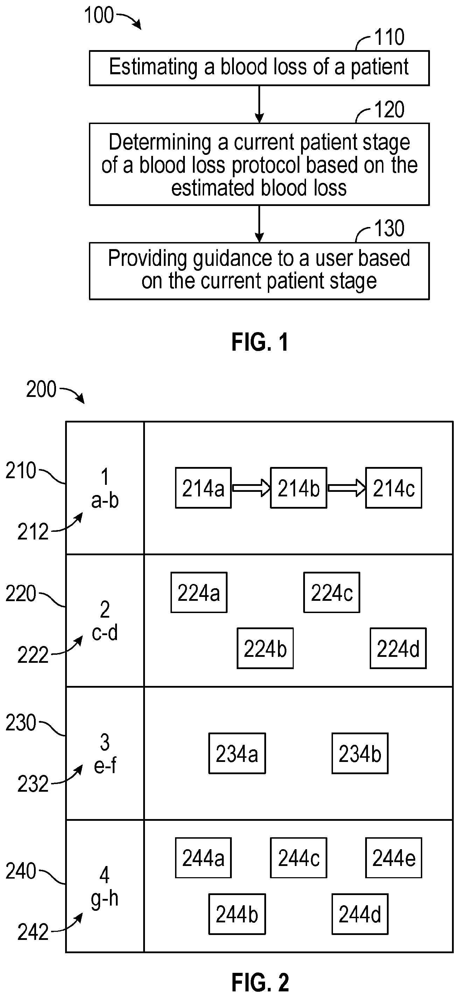

One or more of the example methods described herein may be used to provide guidance to one or more users during a medical procedure. The providing of such guidance may include the use of one or more processors to perform one or more method operations (e.g., method steps). Generally, such methods may include estimating blood loss of a patient and using the estimated blood loss to inform (e.g., generate, select, or otherwise determine) the guidance to be provided to a user. In some example embodiments, such as in the example embodiment depicted in FIG. 1, a method 100 of providing guidance to a user includes estimating 110 the blood loss of the patient, determining 120 a current patient stage of a blood loss protocol based on the estimated blood loss, and providing 130 guidance to the user based at least in part on the current patient stage.

A blood loss protocol (e.g., selected from among various available blood loss protocols) may include a plurality of patient stages, which may be based on the severity of the patient's blood loss, and each patient stage may include one or more elements of that patient stage. In some example embodiments, one or more of the elements of a patient stage may include one or more recommended actions to be performed by the user to manage the blood loss of the patient. In some example embodiments, the estimated blood loss of the patient may be used to determine the current patient stage of the blood loss protocol. Thus, the methods discussed herein may include estimating the patient blood loss. In some example embodiments, the determining of the current patient stage involves comparing the estimated patient blood loss with one or more ranges of blood loss values. Each range may be associated with a different patient stage of the blood loss protocol. Thus, the estimated patient blood loss may be correlated or otherwise associated with a specific patient stage of the blood loss protocol, and this correlation may for the basis for determining the current patient stage of the blood loss protocol (e.g., by comparing the estimated patient blood loss to the blood loss value range for that patient stage, among the various blood loss value ranges associated with the blood loss protocol).

In some example embodiments, one or more of multiple estimated medical parameters may be used (e.g., individually or in combination) to determine the current patient stage in the blood loss protocol. Examples of such medical parameters include blood loss, blood pressure, hematocrit, hemoglobin concentration, concentration of another blood component, heart rate, blood coagulation (e.g., characterized by TEG), temperature, oxygen saturation, height, weight, age, BMI, sex, body fat percentage, intravenous fluid delivered, or any suitable combination thereof. One or more of these medical parameters may be estimated using one or more of the methods described herein (e.g., estimated blood loss or estimated blood component concentration), using suitable instrumentation (e.g., sphygmomanometer, scale, pulse oximeter, thermometer, flow meter coupled to an IV setup, etc.), or any suitable combination thereof. As an illustrative example, if a patient has a high heart rate (e.g., greater than 120 bpm) for a given age (e.g., greater than 70 years), as well as high systolic blood pressure (e.g., greater than 160mm Hg), then a suitable method may include determining the current patient stage for the patient based on the combination of these medical parameters. For example, the current patient stage may be determined by comparing each medical parameter to medical parameter value ranges associated with various patient stages of the blood loss protocol.

In some example embodiments, the providing of the guidance during the medical procedure includes selecting one or more elements from a patient stage (e.g., the current patient stage) of the blood loss protocol. For example, the providing of the guidance may involve selecting one or more elements from the current patient stage of the blood loss protocol. The providing of the guidance may include displaying the guidance (e.g., comprised of the selected one or more elements) to the user via a display screen (e.g. displaying or otherwise presenting the one or more elements on a display screen of a mobile computing device), providing one or more of any suitable notifications (e.g., other notifications such as other visual notifications, audio notifications, tactile notifications, etc.), or any suitable combination thereof.

The guidance provided to the user may be based on (e.g., generated, selected, retrieved, or otherwise determined based on) the current patient stage of the blood loss protocol, a user input, or both. In some example embodiments, the guidance may be provided based on the current patient stage of the blood loss protocol (e.g., where the current patient stage determines the one or more elements to be selected by the processor for inclusion in the guidance to be provided). The guidance may be provided based on the blood loss stage and a user input. For example, the user may indicate that the user has performed a particular action, which may inform the guidance to be provided to the user. In some example embodiments, the methods discussed herein for guiding the user include providing an updated guidance. For example, the guidance may be updated based on a user input, an updated current patient stage (e.g., based on an updated blood loss estimate), or both. The providing of the guidance may include displaying the guidance, the updated guidance, or both, on a display screen, such as on a display screen of a mobile computing device.

### Example Blood Loss Protocols

One or more of the example methods described herein may include the use of a blood loss protocol. For example, a hemorrhage protocol may provide a predetermined set of steps that have been developed based on best practices that have demonstrated desirable results in medical procedures. Hemorrhage protocols may classify the severity of hemorrhage risk into stages (e.g., patient stages) based on the amount of blood the patient has lost. Each stage may provide steps for users to follow to effectively manage the blood loss risk of the patient. One or more of various steps in the blood loss protocol may be provided to the user to guide the user through a series of one or more actions to manage the patient's blood loss. Although hemorrhage protocols may be employed in operating rooms (e.g., labor and delivery suites of a hospital), any suitable blood loss protocol may be used to provide guidance to the user in any medical procedure, in accordance with any one or more of the methods described herein. In some example embodiments, the blood loss protocol may be or include a protocol for managing hemorrhage risk of a patient. In other example embodiments, the blood loss protocol may be related to other types of risk related to blood loss during a procedure (e.g., the medical procedure).

A blood loss protocol, such as a blood loss protocol for managing hemorrhage risk, may be divided into a plurality of patient stages. In some example embodiments, the patient stages of a blood loss protocol are based on the severity of the patient's estimated blood loss. Each stage may be associated with one or more ranges of patient blood loss values, and each range may be defined by an upper threshold blood loss value, a lower threshold blood loss value, or both. FIG. 2 is a schematic depiction of a blood loss protocol 200 divided into four patient stages 210, 220, 230, and 240. Each patient stage has a patient stage number (e.g., 1, 2, 3, 4), based on the severity of patient's blood loss, hemorrhage risk, or both. For example, patient stage 1 may represent the lowest hemorrhage risk, and patient stage 4 may represent the highest hemorrhage risk. One or more patient stages may be defined by a range (e.g., a corresponding range) of blood loss values. In FIG. 2, a patient is classified into patient stage 1 if the estimated blood loss falls within a range 212 defined by threshold values (a) and (b). Likewise, the patient is classified into patient stage 2 if the estimated blood loss falls within a range 222 defined by threshold values (c) and (d). The patient is classified into patient stage 3 if the estimated blood loss falls within a range 232 defined by threshold values (e) and (f). The patient is classified into patient stage 4 if the estimated blood loss falls within the range 242 defined by threshold values (g) and (h). Additionally or alternatively, one or more patient stages may be associated with one or more other suitable patient criteria (e.g., the number of packed red blood cells (PRBCs) that have been administered, vital sign values, etc.) such that the patient may be classified into an appropriate current patient stage based on the estimated patient blood loss, one or more other suitable patient criteria, or any suitable combination thereof.

In some example embodiments, multiple ranges (e.g., more than one noncontiguous range of blood loss values) may be associated with the same patient stage within a blood loss protocol. Different patients, procedures, or conditions may influence the risks presented by different blood loss values. Thus, each patient stage may be associated with a plurality of ranges. Each range may be associated with a medical procedure a patient condition, a patient status, or any suitable combination thereof. For example, each stage may be associated with a first range defined by threshold blood loss values for patients undergoing a vaginal birth, and a second range defined by a second set of threshold blood loss values for patients undergoing a cesarean section. Various ranges associated with each stage may account for any suitable parameter. For example, each stage may be associated with different ranges for men versus women, patients with different BMI classifications, children versus adults, patients who exhibit a particular condition or risk factor versus patients who do not, or any suitable combination thereof.

Each patient stage of a blood loss protocol may include one or more elements of that patient stage. In some example embodiments, each element may be or include a recommended action to be performed by the user to effectively manage patient blood loss. For example, an element of a patient stage of a blood loss protocol may be or include advising the user to: contact a blood bank to request a quantity of blood for a transfusion, begin a blood transfusion, request additional support staff, apply pressure to a wound, suture or wrap a wound, administer an IV, perform a massage or other type of therapy, apply an ointment, administer a drug (e.g., orally or otherwise), or any suitable action. An element of a patient stage may also be or include one or more non-action items, such as notifying the user of the patient's blood type, notifying the user of an allergy of the patient, advising the user of an approximate time that a blood transfusion may be necessary if the patient's blood loss continues at a particular rate, or any suitable combination thereof. An element may also be or include instructions to observe or monitor various parameters, such as one or more vital signs of the patient (e.g., heartrate, oxygen levels, temperature, etc.). FIG. 2 shows elements 214a-214c in patient stage 1, elements 224a-224d in patient stage 2, elements 234a-234b in patient stage 3, and elements 244a-244e in patient stage 4 of the blood loss protocol 200. As depicted in FIG. 2, the number of elements in each patient stage of the blood loss protocol 200 need not be uniform; different patient stages may have different numbers of elements. Further, each patient stage may include any suitable number of elements. In some example embodiments, one or more elements of the blood loss protocol may be ordered in a particular way, such as an order in which the corresponding actions of the elements should be performed. For example, patient stage 1 in FIG. 2 shows elements arranged in a flow chart. In other example embodiments, elements may not be ordered in the blood loss protocol (e.g., in a scenario where the order of the elements depends on a user input). Further, certain elements may appear in multiple stages. That is, each element need not be unique to a particular stage. For example, a blood transfusion may be desirable in both patient stage 3 and patient stage 4. Thus, both patient stages 3 and 4 may include an element that recommends that the user perform a blood transfusion.

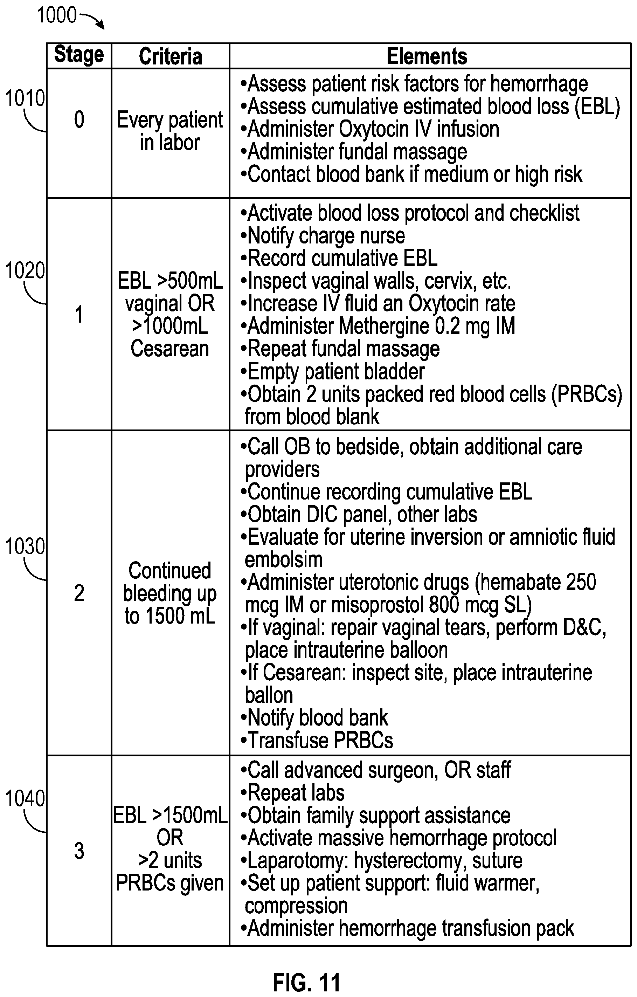

FIG. 11 depicts an example embodiment of a blood loss protocol that may be used to provide guidance to a user. The blood loss protocol shown in FIG. 11 has four patient stages 1010, 1020, 1030, 1040, and each patient stage is associated with one or more ranges of blood loss values, which may be termed "criteria." As discussed above, a range of blood loss values may be associated with more than one patient stage, based on various factors, such as the condition of the patient. For example, patient stage 1 (1020) of the protocol of FIG. 11 is associated with two ranges of blood loss, one for vaginal births (>500mL of blood loss), and one for Cesarean births (>1000 mL of blood loss). Each patient stage of the protocol is associated with a plurality of elements. For example, stage 2 (1030) of the blood loss protocol contains several elements, which are recommended actions that a user may be directed to perform when a patient's blood loss falls within the range associated with patient stage 2 (1020). For example, in patient stage 2, the user is directed to call an obstetrician (OB) to the patient's bedside, obtain additional care providers, administer uterotonic drugs, notify the blood bank, etc. In some example embodiments, not all elements in a given patient stage are appropriate to be performed on every patient. For example, some elements in patient stage 2 are specific to the type of birth being performed (e.g., if vaginal, repair vaginal tears, perform D&C, and place intrauterine balloon; if Cesarean, inspect site, and place intrauterine balloon). The blood loss protocol depicted in FIG. 11, however, is merely illustrative and includes examples of the kinds of criteria, elements, or both, that may be included in a blood loss protocol.

One or more blood loss protocols may be stored in the memory of one or more devices. In some example embodiments, one or more blood loss protocols are stored in the memory of a mobile computing device, such as a smartphone, laptop, or tablet. In some example embodiments, one or more blood loss protocols are stored in the memory of a remote device, and may be wirelessly accessible. For example, a blood loss protocol may be stored in a cloud-based storage system. A user may upload the blood loss protocol into the memory of a computing device in any suitable manner. In some example embodiments, the blood loss protocol is customizable. For example, one or more user settings may allow a user to adjust one or more parameters of the blood loss protocol. In some example embodiments, the user may be able to set threshold values for the ranges or for other patient criteria associated with one or more patient stages of the blood loss protocol. As another example, one or more user settings may allow the user to adjust one or more elements of the blood loss protocol (e.g., customizable set of recommended actions for one or more patient stages). Such user settings may enable, for example, a hospital or practitioner to customize the blood loss protocol for their purposes (e.g., in general as a default protocol, for a specific patient demographic, for a specific procedure type, etc.). In some example embodiments, the blood loss protocol may automatically adjust (e.g., adjusted by suitable software executing on a suitable device) based on patient information such as height, weight, sex, medical history, procedure information, or any suitable combination thereof. Information regarding the patient, the medical procedure, or any other suitable information used to customize a blood loss protocol may be inputted manually by the user, or may be received by other means (e.g., electronic or wireless transmission). Further, updated versions of the blood loss protocol may be downloaded onto a server and into memory, either automatically or by the user.

### Examples of Estimating Blood Loss

One or more of the example methods described herein for guiding a user may include estimating a blood loss of a patient. Various example methods may be used to estimate the amount of blood lost by the patient during a medical procedure. In some example embodiments, two or more of such methods may be combined to arrive at an estimated patient blood loss. In some example embodiments, one or more image analysis techniques are used to estimate the patient blood loss.

For example, as further described below, estimating blood loss may include analyzing an image depicting one or more fluid-containing substrates, such as fluid containers, surgical items (e.g., surgical textiles, such as surgical sponges), or both. The estimating of blood loss may include extracting a feature from an image, such as a color parameter, and associating the feature with an estimated blood component concentration. The estimated blood component concentration may be used to determine a fluid volume in a fluid- containing substrate. The volume of fluid in the substrate may be used to determine total patient blood loss, either alone or in combination with the estimated fluid volume in other fluid-containing substrates (e.g., among multiple such substrates). As another example, as further described below, the estimating of blood loss may alternatively or additionally comprise utilizing weight information to determine an estimated blood loss of a patient. For example, the estimating of blood loss may comprise weighing one or more fluid containing substrates (e.g., surgical items, fluid containers, etc.), and associating the weight information with an amount of blood lost by the patient. In some example embodiments, the weight information may be combined with information derived from one or more image analysis techniques to a determine the estimated patient blood loss. As yet another example, one or more manual methods of estimating the blood loss may be used, such as using one or more containers with fluid level markings. Accordingly, one or more of the example methods discussed herein may comprise analyzing multiple fluid containers, surgical items, or both, to estimate patient blood loss.

### Example Image Analysis Techniques for Estimating Blood Loss

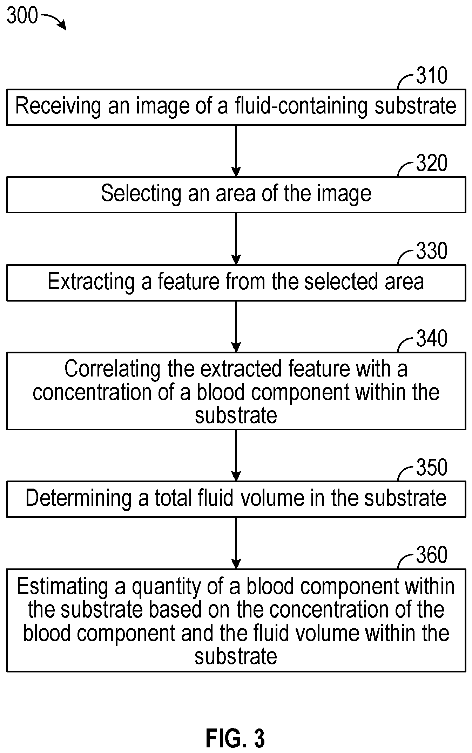

In some example embodiments, the estimating of the blood loss of the patient includes the use of one or more image analysis techniques to determine the amount of blood in one or more fluid-containing substrates, such as one or more fluid containers, surgical items (e.g., surgical textile or sponge, etc.), or any suitable combination thereof. For example, the estimating of the patient blood loss may include one or more of the methods described in U.S. Pat. No 8,792,693 and U.S. Pat. No 8,983,167. Generally, as depicted in the schematic block diagram in FIG. 3, a method 300 for estimating of patient blood loss using one or more images that depict one or more fluid-containing substrates may include receiving 310 an image of a fluid containing substrate, selecting 3320 an area of the image containing at least a portion of the fluid-containing substrate, extracting 330 a feature from the selected area, correlating 340 the extracted feature with a concentration of a blood component within the substrate, and estimating 350 a quantity of the blood component within the substrate based on the concentration of the blood component within the substrate.

For example, as shown in FIG. 3, the method 300 of estimating of the blood loss may include receiving 310 an image of a fluid-containing substrate. The image may be static, video (e.g., a color frame of a live video feed), infrared, a field of view of an optical sensor, black and white, a fingerprint of a field of view of an optical sensor, a point cloud, any other suitable type of image, or any suitable combination thereof. Any suitable device may be used to generate the image, receive the image, perform any subsequent image processing, or any suitable combination thereof. For example, a camera on a mobile computing device may be used to generate the image of the fluid- containing substrate. A processor (e.g., a processor of a mobile device) may receive the image, perform image processing and analysis, or both, to estimate the blood loss. The image may depict all or a portion of the fluid-containing substrate, and the image may depict multiple fluid containing substrates. The example method depicted in FIG. 3 includes selecting 320 an area of the image. The selecting of the area of the image may include selecting an area of the image corresponding to the fluid-containing substrate. For example, one or more area selection methods may utilize edge detection techniques, machine learning, or both, to identify and isolate the fluid containing substrate in the image. The selecting of the area of the image may comprise identifying a portion of the image containing blood or other fluid. For example, the selecting of the area of the image may include detecting a fluid level in a canister or other fluid container and isolating the portion of the image that corresponds to the fluid, detecting and isolating a fluid-saturated portion of a surgical item such as a surgical textile, or any suitable combination thereof.

The example method 300 depicted in FIG. 3 also includes extracting 330 a feature from the selected area of the image. The extracted feature may be a feature indicative of a blood component concentration in the fluid-containing substrate. The extracted feature may be a color (e.g., red), a color intensity (e.g., redness value), a luminosity, a hue, a saturation value, a brightness value, a gloss value, or other color- related value in one or more component spaces, such as the red, blue, green, cyan, magenta, yellow, key, or lab component spaces. In some example embodiments, the extracting of the feature may include extracting one or more features that make generate a histogram of various color or color-related values in a set of pixels within the selected area of the image.

The example method 300 depicted in FIG. 3 further includes correlating 340 the extracted feature with a blood component concentration of a portion of the fluid-containing substrate. Generally, the correlating of the extracted feature with the blood component concentration may include correlating the extracted feature to a value that indicates an amount of blood in the fluid-containing substrate. For example, the correlating of the extracted feature to the value may include transforming one or more features (e.g., color features) extracted from the image into an estimated concentration of a blood component within the fluid-containing substrate (e.g., a fluid canister or a surgical item). The blood component can be any of whole blood, red blood cells, hemoglobin, platelets, plasma, white blood cells, or any other blood component. For example, the transforming of one or more features into the estimated concentration of the blood component may implement or otherwise be based on one or more parametric analysis techniques, non-parametric analysis techniques, such as those described in U.S. Pat. No. 8,983,167 and U.S. Pat. No. 8,792,693, respectively, or any suitable combination thereof, to estimate the concentration of the blood component within the fluid in the fluid-containing substrate (e.g., the fluid canister).

As shown in FIG. 3, the example method 300 involves extracting 330 one or more features (e.g., from pixel clusters) within the selected area of the image. In some example embodiments, this is followed or otherwise accompanied by tagging each pixel cluster with a blood volume indicator based on a non-parametric correlation of each pixel cluster with a template image in a library of template images of known blood component concentration. For example, the estimating of the blood component concentration may involve extracting a color intensity in the red component space from a set of pixel clusters, and implementing a K-nearest neighbor method to compare each extracted feature with redness intensity values of template images. Each template image can include a pixel cluster tagged with a known fluid quality, such as hemoglobin volume or mass per unit volume of fluid or per pixel unit (e.g., hemoglobin concentration). Each template image can include a pixel cluster tagged with the volume, mass, density, etc. per unit volume of fluid or per pixel unit of any other liquid or solid in the canister. Once a suitable match between a particular pixel cluster and a particular template image is identified, the estimating of the concentration of the blood component may include projecting known fluid quality information from the particular template image onto the particular pixel cluster. Thus, the selected image region, pixel, pixel cluster, or any suitable combination thereof, may be paired with the closest- matching template image identified with the K-nearest neighbor method, and the selected image region, pixel, pixel cluster, or combination thereof, may be estimated as depicting the blood component concentration associated with the closest-matching template image. The estimating of the blood component concentration may also aggregate, average, or otherwise combine pixel cluster tags to estimate output a total blood component concentration for the fluid in the fluid-containing substrate. However, various example embodiments of the methods discussed herein may include correlating the extracted featured with a concentration of a blood component within the substrate via any other suitable non-parametric method or technique.

In another example, absolute differences in pixel intensities (e.g., in red, green, or blue intensities or color values, or any suitable combination thereof) may be used for template matching. Such an absolute difference in pixel intensities may be calculated at a wavelength of light that correlates with the blood component (e.g., at about 400 nm for estimating hemoglobin concentration). More specifically, a sum of absolute differences in pixel intensities may be used to compare pixel intensities between the receptacle image region and each template image. The closest-matching template image is identified when the sum of absolute differences is substantially minimal compared to other sums of absolute differences calculated for an image region and other template images. Thus, the image region may be paired with the closest-matching template image identified with the sum of absolute differences method, and an image region (e.g., a pixel cluster) may be estimated as depicting the same blood component concentration associated with the closest-matching template image.

In yet another example, the estimating of the blood component concentration may include extracting 330 features from pixel clusters within the selected area of the image, and implementing a parametric model or function to tag each pixel cluster with a blood component concentration. As described in U.S. Pat. No. 8,792,693, the estimating of the blood component concentration may include inputting (e.g., inserting) one or more extracted features from one pixel cluster into a parametric function to substantially directly transform the one or more extracted features from the pixel cluster into the blood component concentration. This process may be repeated for each other pixel cluster in the selected area. As described above, the one or more extracted features may include any one or more of a color intensity in the red component space, a color intensity in the blue component space, a color intensity in the green component space, or any suitable combination thereof. The parametric function may be or include a mathematical operation or algorithm that relates color intensity to hemoglobin mass per unit fluid volume. Generally, color values of the template images may be used to train or generate a parametric model (e.g., in the form of a mathematical function, curve, or algorithm) that correlates a pixel color value to a blood component concertation.

Reflectance of oxygenated hemoglobin (HbC) at certain wavelengths of light can be indicative of the concentration of hemoglobin per unit volume of fluid. Therefore, in some example embodiments, an extracted feature may be or include one or more reflectance values at a particular wavelength for each pixel cluster in a set of pixel clusters in the selected area of the image. The correlating of the extracted feature with a concentration may include converting each reflectance value into a corresponding hemoglobin concentration value by implementing a parametric model. The hemoglobin concentration values may be combined to estimate the total (e.g., average) hemoglobin concentration in the fluid-containing substrate. Furthermore, because the hemoglobin content of a wet (e.g., hydrated) red blood cell is typically about 35%, the red blood cell concentration can be extrapolated from the hemoglobin concentration based on a static estimated hemoglobin content (e.g., 35%). Furthermore, the extracting of the feature from the selected area may include accessing a recent measured hematocrit or estimate a current hematocrit of the patient (e.g., as described in U.S. Pat. No. 9,936,906), and the correlating of the extracted feature may utilize the measured or estimated hematocrit to transform the estimated red blood cell concentration into an estimated extracorporeal blood concentration. However, the correlating of an extracted feature with a concentration of blood component can implement any other parametric analysis, non-parametric analysis, or both, of single pixels or pixel clusters within the selected area to estimate the concentration of any one or more blood components in fluid within the fluid-containing substrate (e.g., the fluid canister).

The estimating 350 of the quantity of the blood component in the fluid-containing substrate may include determining a total volume of fluid contained within the substrate. In some example embodiments, information about the weight of the substrate, the fluid level in the substrate, the dimensions of the substrate, or any suitable combination thereof, may be used to determine a fluid volume within the substrate. For example, weight information may be combined with a known approximate density of a fluid in the substrate to arrive at a fluid volume of fluid within the substrate. In another example, the fluid level of the substrate in the example form of a canister, in combination with the dimensions of the canister, may be used to determine the volume of fluid in the canister. In another example, dimensions of a surgical item (e.g., a surgical textile) may be used determine a surface area of the surgical item, which may be combined with information about the saturation level and blood component concentration to determine the volume of fluid in the surgical item.

In some example embodiments, the estimating of the blood content in the fluid-containing substrate includes estimating the blood content based at least in part on a fluid level in a container (e.g., a fluid canister), which may form all or part of the fluid-containing substrate. For example, the fluid level in the container may be used to determine the volume of fluid in the container, which may be used to calculate an amount of blood in the container (e.g., in combination with a blood component concentration). In some example embodiments, the fluid level or volume of fluid in the container may be determined by receiving such information through a user input. Additionally or alternatively, fluid level or volume of fluid may be estimated by one or more processors. The estimating of the fluid level in the container may involve correlating a portion of the selected area of the image with a fluid level within a canister. For example, the estimating of the fluid level may include identifying a surface of fluid in the canister and a base of the canister (e.g., a lowest extent of fluid in the canister) and, from this data, estimating the level of fluid within the canister. In some example embodiments, a processor may fit a parametric function (e.g. sigmoid) to an intensity profile of the selected area that corresponds to an anti-glare strip on the canister to estimate a fluid height therein. In other example embodiments, a processor may correlate pixels in the selected area with fluid (e.g. pixels that are substantially red) and calculate an upper bound and a lower bound of fluid in the canister based on the distribution of y-coordinates of the correlated pixels. In such example embodiments, the processor can define the upper bound as the 95th percentile of the y-coordinates, or the processor can begin with the 99th percentile of the y-coordinates of the correlated pixels and decrement the percentile until the redness of two adjacent pixels does not change beyond a predetermined threshold, though the process may function in any other way to identify, ignore, or both, any "false-positive" "red" pixels that do not correspond to fluid in the canister.

In some example embodiments, the estimating of the blood content in the fluid containing substrate includes estimating the blood content based at least in part on the estimated volume of blood in the fluid- containing substrate, such as a surgical item (e.g., a surgical textile), by generating information about the dimensions of the surgical item (e.g., an estimated surface area of the surgical item, an estimated surface area of the soiled portion of the surgical item, a pixel count of a portion of the surgical item, a pixel count of the soiled portion of the surgical item, or any suitable combination thereof). In some example embodiments, the estimating of the blood content in the fluid-containing substrate may implement object recognition to isolate an object within a field of view of an optical sensor, within the image, or both. The object may have known dimensions, or the dimensions of the object may extrapolated, such as by estimating the distance from the optical sensor, the angle between the optical sensor and the object with known dimensions, or both, and comparing the position of the fluid-containing substrate and the object with known dimensions in the image. In other example embodiments, the determining of the dimensions of the fluid-containing substrate may involve determining the number of pixels along an axis of the fluid-containing substrate, and using information about the distance from the optical sensor to the fluid-containing substrate to establish a relationship between number of pixels and a numerical dimension.

The estimating 360 of the quantity of blood within the fluid- containing substrate may utilize the concentration of the blood component within the fluid-containing substrate (e.g., correlated or otherwise determined based on the extracted feature), and the determined total fluid volume in the substrate to estimate the quantity of blood component within the fluid-containing substrate. Generally, the estimating of the quantity of the blood component may include calculating a quantity (e.g., mass, weight, volume, cell count, etc.) of the blood component by multiplying the estimated volume of fluid in the fluid-containing substrate by the estimated concentration of the blood component in the fluid in the fluid-containing substrate (e.g., the canister). Furthermore, the estimated blood loss (e.g., blood volume lost) may be estimated by dividing the estimated quantity of the blood component (e.g., hemoglobin) by a value for a serum blood component mass value that may, for example, be laboratory-derived and entered by a user.

The estimating of the patient blood loss may also include combining blood volume estimates from multiple substrates. For example, the estimating of the total patient blood loss may include summing estimated blood volumes in multiple fluid-containing substrates. The estimating of the patient blood loss may include combining blood quantity estimates from the same type of fluid-containing substrates (e.g., two or more fluid canisters), or different types of substrates (e.g., combine the canister blood volume estimate with estimated blood volumes in surgical items, such as surgical gauze sponges, surgical towels, surgical drapes, surgical dressings, or any suitable combination thereof). The example methods discussed herein may include adding estimated patient blood loss data to a medical record of a patient, triggering an alarm once a threshold extracorporeal blood volume estimate is reached, or estimating a current patient hematocrit based on initial patient hematocrit, fluid IVs, transfusions, and total estimated blood loss, such as described in U.S. Pat. No. 9,936,906.

### Other Examples of Estimating Patient Blood Loss

Some methods of estimating patient blood loss may not utilize image analysis. However, as described above, one or more techniques involving image analysis may be combined with other techniques to estimate patient blood loss.

In addition to the use of weight information described above (e.g., combining weight information with a blood component concentration derived from an extracted image feature to estimate blood content of a substrate), weight information may be used without the use of image analysis to estimate patient blood loss. For example, the weight of one or more fluid-containing substrates may be used to estimate a quantity of blood contained in the one or more fluid- containing substrates using pre -determined information about the blood content of the one or more fluid-containing substrates. In some situations, it may be generally assumed that fluid contained in a substrate is all or substantially all blood (e.g., human blood). A known density of such blood may be used to correlate the weight of the fluid in the one or more fluid-containing substrates (e.g., as estimated as the difference between a "wet" weight and a "dry" weight of a fluid-containing substrate) to a fluid volume. In other situations, a blood component concentration in a fluid may be known with a reasonable level of certainty without the use of image analysis, and the blood component concentration may be used to estimate an amount of blood in a fluid-containing substrate. Any suitable weight detection system or method may be used to determine the weight of a substrate configured to contain fluid. For example, one or more of such methods may include placing an item, such as a fluid container or a surgical textile, on a scale to determine the weight. In some example embodiments, weight information (e.g., indicting the weight) may be received by a processor to estimate a quantity of blood contained in fluid-containing substrate based on the weight information. The weight information may be automatically transmitted to the processor (e.g., wirelessly transmitted from the scale) or may be entered manually by a user (e.g., into a computing device such as smartphone, tablet, laptop, etc.).

In some example embodiments, one or more manual techniques may be used to estimate patient blood loss. For example, blood may be collected into a fluid canister, and volume indicators (e.g., markings) on the canister may be used to estimate the amount of blood in the canister. A fluid canister may be placed near the patient in a position such that blood lost by the patient may be drained into the canister. A user may observe one or more markings on the canister, and the one or more markings may indicate the volume of fluid in the canister when the fluid reaches the level of each marking. In some example embodiments, the user manually enters the volume (e.g., as indicated by the fluid level), into a processor (e.g. a processor of a mobile computing device). In other example embodiments, one or more imaging systems, fluid level sensors, or any suitable combination thereof, determine when fluid has reached a particular level of the canister and automatically transmit fluid level information to the processor.

U.S. Pat. Pub. No. 2018/0199827 describes examples of non-image based methods of quantifying fluids that are lost by a patient during a medical procedure (e.g., during labor and delivery, a surgical procedure, etc.). One or more of various methods, systems, or both, may be used to track or otherwise estimate a quantity of fluid (e.g., blood) lost by the patient throughout the medical procedure, and the estimate may be updated and displayed in substantially real-time during the medical procedure, at the conclusion of the medical procedure, or both. During the medical procedure, extracorporeal fluids (e.g., blood) that are lost by the patient may be collected with surgical items. Examples of such surgical items include surgical textiles or other absorbent surgical items, such as surgical sponges (e.g., laparotomy sponges), surgical dressings, surgical gauze, surgical towels, absorbent pads or drapes (e.g., chux pads), vaginal packs, other textiles, other absorbent items, or any suitable combination thereof. One or more surgical textiles or other absorbent surgical items may be placed in a bag (e.g., sponge counting bag) for tracking purposes, hygienic purposes, or both. Additionally or alternatively, extracorporeal fluids that are lost by the patient may be collected in a container (e.g., a fluid container), such as a canister (e.g., a fluid canister). Some applications of such methods and systems may involve collection of lost fluids with a specialized container. For example, during labor and delivery procedures, a drape with at least one pocket (e.g., a blood collection drape with a triangular pocket) may be placed under the patient for collecting blood, amniotic fluid, urine, or any suitable combination thereof. The quantity of fluid collected in at least some types of surgical items, such as surgical textiles or fluid canisters, may be estimated based on a measured weight (mass) of the surgical item when containing fluid, or may be measured based on volume indicators or markings on the surgical item (e.g., on the fluid canister). In some implementations, multiple batches of such items are weighed (e.g., as each batch of one or more surgical textiles become saturated) and aggregated into a running total or overall estimate of the quantity of fluid lost by the patient during the medical procedure. Such an estimate may be combined with estimates of fluid collected in batches or cumulatively overtime in some other types of surgical items, such as canisters or blood collection drapes. For example, the total volume of fluid, the total rate of fluid loss, or both, may be estimated at any particular point during the medical procedure, post-procedure, or both.

### Examples of Determining a Current Patient Stage of a Blood Loss Protocol

Guiding a user during a medical procedure may include determining a current patient stage of a blood loss protocol. The determining of the current patient stage may be based on estimated patient blood loss. As described above, one or more of various methods may be used to determine the estimated patient blood loss. The determining of the current patient stage of the blood loss protocol may include comparing the estimated patient blood loss with threshold values of blood loss ranges associated with each patient stage of the blood loss protocol, assessing the patient against other patient criteria (e.g., rate of transfusions, vital signs, or both), or any suitable combination thereof. In some example embodiments, each patient stage of the blood loss protocol is associated with more than one range. Thus, the determining of the current patient stage may also include selecting the appropriate range against which the estimated patient blood loss is to be compared. Furthermore, as described above, multiple medical parameters may be used to determine the current patient stage of the blood loss protocol.

In some example embodiments, a processor may receive the estimated patient blood loss, access the blood loss protocol stored in the memory of a device, and compare the estimated patient blood loss with the threshold values associated with each range of the blood loss protocol to determine the current patient stage. The processor may then communicate the current patient stage to one or more subsequent method steps, such that one or more processors may utilize the current patient stage to provide guidance to the user. In some example embodiments, the estimated patient blood loss is automatically transmitted to a processor. For example, in implementations where the patient blood loss is estimated using one or more computational methods, such as those described above (e.g., image analysis, analyzing weight information, etc.), the estimated blood loss of the patient may be automatically transmitted to, and received by, a processor to be utilized in providing guidance to the user. Components of a blood loss estimation system, and components used to determine the current patient stage, may be in communication with each other in any suitable manner. For example, a transmitter/transceiver of a blood estimation system may communicate with a transmitter/receiver of a system to determine the current patient stage (e.g., wirelessly or through wired communication). In some implementations, both blood loss estimation and the determination of a current patient stage may be carried out on the same system, the same processor, or both. In some example embodiments, the determining of the current patient stage comprises accessing data pertaining to the estimated blood loss from the memory of one or more devices.

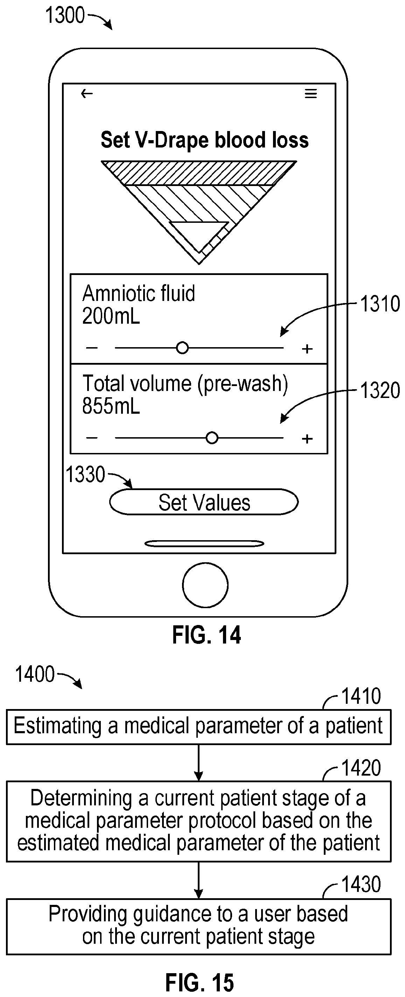

In other example embodiments, the estimated patient blood loss may be manually entered by a user, adjusted or updated by the user, or both. For example, where the patient blood loss is estimated using one or more manual techniques, such as by using markings on a fluid container to determine blood volume, using weight information to estimate blood loss, or both, the user may wish to manually enter the patient blood loss into a system for use in determining the current patient stage. Further, in some example embodiments, the user may wish to adjust or update an automatically transmitted patient blood loss. For example, it may be the case that the patient blood loss was determined by a processing system utilizing images of fluid canisters, surgical textiles, or both, but additional blood loss information was not captured by the system. Therefore, the user may then wish to add an additional amount of blood loss to the estimate. FIG. 14 shows an example of a GUI 1300 that allows the user to manually enter a blood loss value from a fluid container or enter fluid volume values from which a blood loss value may be derived. The GUI 1300 in FIG. 14 allows the user to adjust the volume of a first fluid (e.g., amniotic fluid) in the fluid container (e.g., a V-Drape) using one slider icon 1310, and to adjust the total volume of the fluid in the fluid container using another slider icon 1320. For example, the position of a marker along the slider icon 1310, the slider icon 1320, or both, may be adjusted by the user clicking and dragging the marker itself, by selecting incrementing or decrementing buttons, or both. In the depicted example, the blood loss value may be determined as the difference between the total fluid volume entered via the slider icon 1320 and the amniotic fluid volume entered via the slider icon 1310 (e.g., assuming that the two primary fluids in the fluid container are amniotic fluid and blood). When the user is finished adjusting the values for each metric, the user may press the "Set values" button 1330 to store the information inputted by the user, the fluid volume (e.g., blood loss value) derived therefrom, or both. Although FIG. 14 shows the GUI 1300 allowing the user to enter the amount of fluid contained in a V-drape, a similar display may be used to allow the user to enter the amount of fluid (e.g., blood) in any type of container. Similarly, although the GUI 1300 includes slider icons (e.g., slider icons 1310 and 1320) to allow adjustment of fluid volumes, it should be understood that other mechanisms of input (e.g., typing in a numerical value, recitation of a number that is parsed using voice recognition techniques, etc.) may be suitably used.

By way of example, a processor may receive an estimated patient blood loss value for patient A of 1200 mL of blood. A blood loss protocol stored in memory of a computational device may include three (3) patient blood loss stages. Patient stage 1 of the blood loss protocol may be associated with the range 500mU-1000mU; patient stage 2 may be associated with 1000 mU-1500 mL; and patient stage 3 may be associated with >1500 mL. Thus, one or more methods of determining the current patient stage may include comparing the estimated blood loss of Patient A with the threshold values defining the range associated with patient stage 1, the range associated with patient stage 2, and the range associated with patient stage 3. Based on the comparison, the processor would determine that the estimated blood loss of patient A of 1200 mL falls between the threshold values of 1000 mL and 1500 mL, and therefore the current patient stage should be patient stage 2. It should be understood, however, that these blood loss value ranges are only examples, and other threshold blood loss values may be used to determine which stage is the current patient stage.

### Examples of Providing Guidance

One or more of the methods discussed herein may include providing guidance to a user based on one or more of the current patient stage of the blood loss protocol, one or more user inputs, or any suitable combination thereof. The providing of such guidance may include selecting one or more elements from the current patient stage of the blood loss protocol. As described above, each patient stage may include one or more elements that may be selected by a processor. In some example embodiments, one or more of the elements of the current patient stage of the blood loss protocol may be or include one or more recommended user actions.

### Examples of Determining Guidance to be Provided to a User

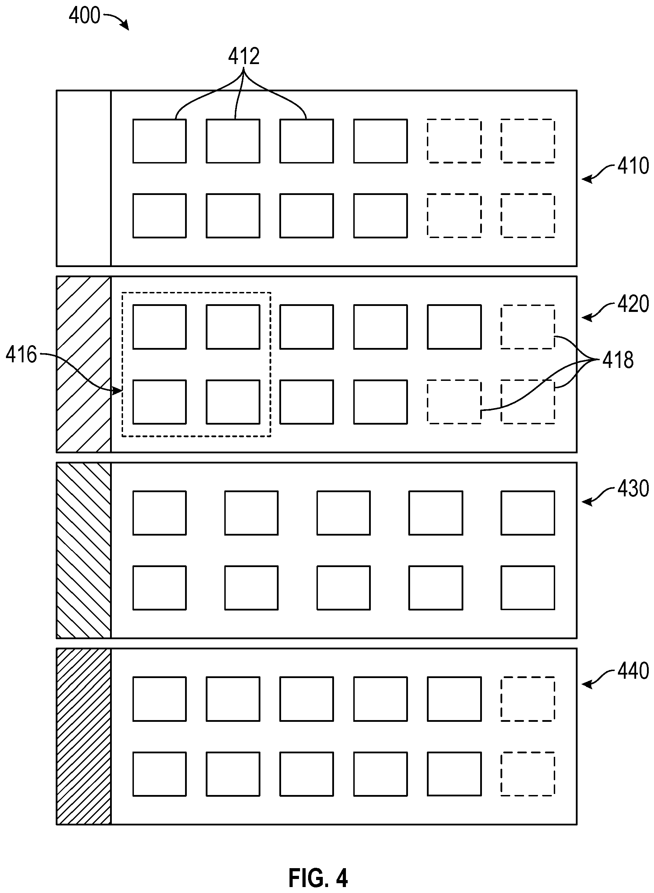

FIG. 4 is a schematic depiction of a method 400 of providing guidance to a user. Generally, providing guidance to a user includes selecting 412 one or more elements from a patient stage (e.g., patient stages 410, 420, 430, or 440) for providing as the guidance. In the example embodiment shown in FIG. 4, the guidance 416 includes four elements selected from the patient stage 420. However, any suitable number of elements may be selected to be provided as the guidance. In some example embodiments, one or more elements to be included in the guidance are selected based on the current patient stage. In other example embodiments, one or more elements to be included in the guidance are selected based on the current patient stage in combination with user input. For example, such user input may include information about the patient (e.g., parameters such as height, weight, sex, physical conditions, medical history, family history, pre-operative platelet count, pre-operative hematocrit, pre operative hemoglobin, vitals, or any suitable combination thereof), the medical procedure being performed on the patient, the hospital, the doctor, one or more actions the user has already performed, or any suitable combination thereof. For example, a user input may indicate that the patient is obese, and therefore at a higher risk of a blood-loss related complication.

Based on this information about the patient, and based on the current patient stage, the guidance may include an element that recommends that the user prepare to give a blood transfusion. User input may be received in any suitable manner, such as the user interacting with a mobile computing device (e.g., pressing one or more buttons on the mobile computing device to indicate completion of an element of the blood loss protocol.). In some example embodiments, information regarding the patient, the medical procedure, the hospital, one or more doctors, one or more actions performed by the user, or any suitable combination thereof, may be automatically transmitted to and received by one or more processors. Thus, "user input" may be data automatically transmitted based on an action performed by the user, information obtained by the user, items manually entered by the user, or any suitable combination thereof.

In some example embodiments, one or more patient stages may include one or more elements that are conditionally selectable for providing as guidance. For example, one or more elements may be selectable upon an indication that the user has performed one or more particular actions, or that one or more particular events have occurred (e.g., with the one or more elements not being selectable prior to such performance or occurrence). In other words, the particular one or more actions or events may "unlock" the one or more conditionally selectable elements and enable these one or more unlocked elements to be provided as guidance to the user thereafter. Conditionally selectable elements 418 are depicted schematically in FIG. 4 by dashed lines. However, upon the occurrence of one or more events, a user input, or both, the one or more conditionally selectable elements may be reclassified as selectable. Classifying some elements as unselectable (e.g., non-selectable or otherwise not selectable) until or unless a particular event occurs may be desirable if those elements are relevant in limited circumstances. For example, some elements may only be relevant once the user has indicated that they have started a blood transfusion, administered an IV transfusion, or started to suture a wound. Similarly, one or more patient stages may include elements that are conditionally unselectable (e.g., the element is disabled such that the functionality associated with the element is not performed despite a user selecting the element) for providing as guidance. For example, one or more elements may be unselectable or hidden upon an indication that the user has performed a particular action, or that a particular event has occurred (e.g., with the one or more elements being selectable before this action or event as occurred). In other words, the particular action or event may "lock" the one or more conditionally unselectable elements and disable these one or more locked elements to thereby prevent them from being provided as guidance to the user thereafter. Thus, depending on one or more predetermined triggers (e.g., actions, events, or any suitable combination thereof), one or more elements may be unlocked (e.g., becoming providable as guidance) or locked (e.g., becoming not providable as guidance).

In some example embodiments, the providing of guidance to the user includes providing updated guidance to the user. Updated guidance may be provided based on any suitable indication or event, such as, a user input, an updated current patient blood loss stage, or any suitable combination thereof. The updated guidance may be provided based on a user input. For example, the user may indicate that the user has performed a particular action, that patient has exhibited a certain condition, or both, which may prompt an updated list of elements to be selected for provision to the user and then provided to the user. In some example embodiments, updated guidance may be provided based on an updated patient stage of the blood loss protocol. For example, the estimating of blood loss may include continuously or periodically estimating the patient blood loss (e.g., the blood loss of the patient undergoing the medical procedure). An updated estimate of the patient blood loss may therefore be determined at various time points during the medical procedure, and an updated patient stage may be determined based on the updated patient blood loss. Thus, updated guidance may be provided to the user based on the updated patient stage, as updated based on the updated blood loss. In some example embodiments, update guidance may be provided to the user or to the patient, for example, based on both user input and the updated patient stage (e.g., based on the updated blood loss).

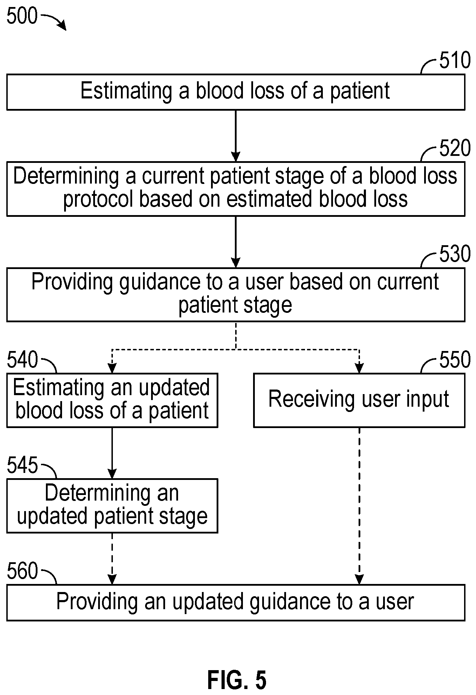

FIG. 5 is a schematic depiction of a method 500 of providing guidance to a user, where the providing of guidance includes providing 530 initial guidance and providing 560 updated guidance. As described above, the method 500 of providing guidance to the user may include estimating 510 a blood loss of a patient, determining 520 a current patient stage of a blood loss protocol based on the estimated blood loss, and providing 530 guidance to the user based on the current patient stage. As represented by the dashed arrows pointing from the providing 530 of the guidance, providing updated guidance may be based on one or more types of information. Providing updated guidance may include estimating 540 an updated blood loss of the patient, and determining 545 an updated patient stage of the blood loss protocol based on the updated blood loss. In some example embodiments of the method 500, the providing 560 of the updated guidance includes receiving 550 a user input. Thus, the providing 560 of the updated guidance may be based on a user input, an updated patient stage (e.g., updated based on an updated blood loss of the patient), or both.

### Examples of Providing the Determined Guidance

Providing guidance (e.g., including any updated guidance) may include displaying or otherwise presenting the guidance, other information, or both, to the user. For example, the providing of the guidance to the user may include displaying one or more elements selected by a processor onto a display (e.g., display screen). The display may be a display screen that is part of a mobile computing device, such as a tablet, a laptop, a smartphone, or any suitable computational device. As another example, the display may be part of desktop computing console or the like. Additionally or alternatively, the providing of the guidance to the user may include displaying guidance on a projection surface (e.g., wall, projector screen, etc.). Guidance may be displayed proximate to the patient (e.g., in the same room), outside the medical procedure room, such as near a medical department desk (e.g., a nurses' desk), a lounge, a common area, etc. such that medical staff outside of the immediate vicinity of the patient may be kept aware of the provided guidance, the patient's status (e.g., in substantially real-time fashion), or both.

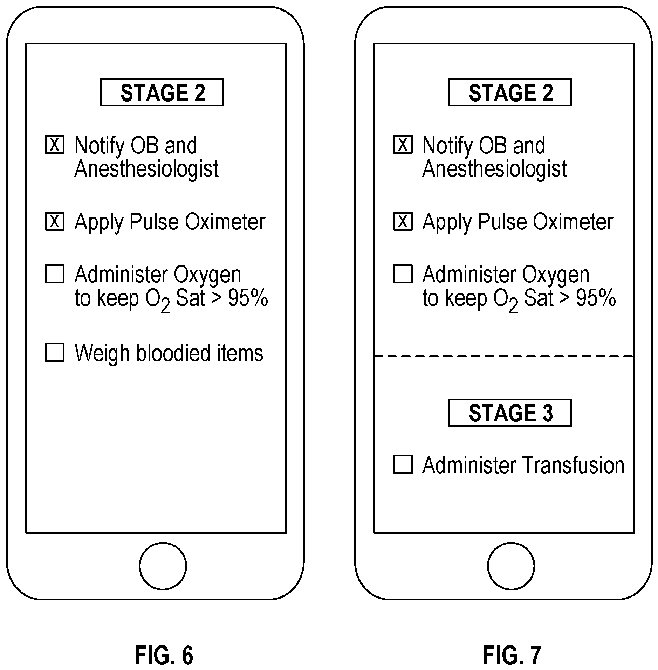

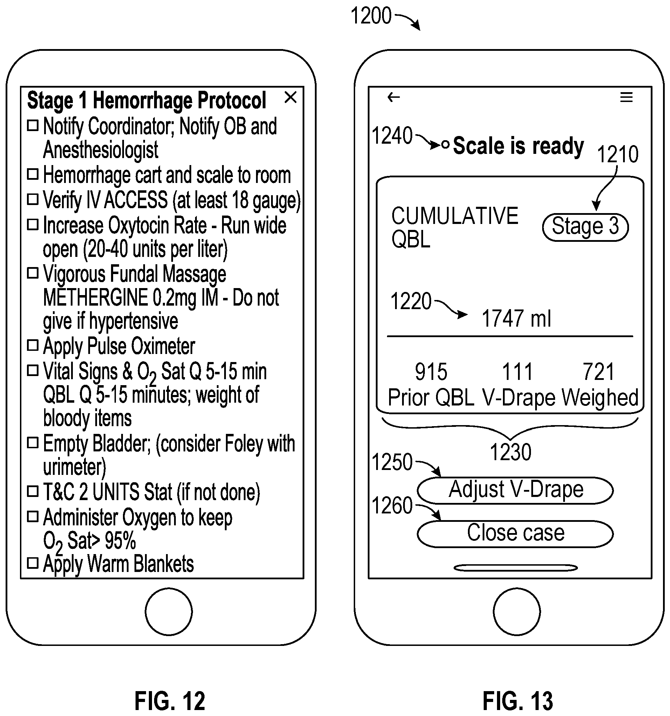

FIG. 6 depicts an example of a GUI for a display screen of a mobile device displaying various elements of a blood loss protocol. The example GUI of FIG. 6 depicts a series of elements provided to the user for the current patient stage, which in FIG. 6 is "stage 2." As depicted in FIG. 6, the display may provide one or more elements that include one or more recommended actions for the user to complete. The display may provide one or more elements that are complete, incomplete, or any suitable combination thereof, and may include an indication of whether an action has been completed. For example, the display may include a box next to each element, such that completed elements may be indicated by a marking. FIG. 6 shows boxes with completed items indicated by an "x," but any suitable marking may be used (e.g., a check mark, a slash, a star or asterisk, filling in the box, crossing out the text of the element, etc.). As described above, one or more elements may be marked as completed, automatically or via a user input (e.g., by manual user input through a touch screen, by recited user input that may be parsed and interpreted, etc.). In some example embodiments, the display presents elements in one or more of various colors to indicate whether they have been completed (e.g., green items have been completed, while red items need to be completed). The display may adjust a color intensity based on whether an element has been completed (e.g., with incomplete elements listed in a darker color or in black, while completed elements are lightened or greyed out). FIG. 12 depicts another example GUI (e.g., for a display screen of a mobile computing device) displaying various elements of a blood loss protocol. The GUI in FIG. 12 depicts a series of example elements provided to the user for a patient stage, labeled as "stage 1," of a blood loss protocol.

Displayed elements of the guidance may be arranged in any suitable manner. In some example embodiments, some or all elements may be grouped into categories and displayed in conjunction with one or more of various headers. For example, some or all elements may be grouped into categories, such as assessments (e.g., assess cumulative blood loss, evaluate vaginal wall, evaluate cervix, evaluate placenta, evaluate uterine cavity, measure patient vitals, etc.), medications (e.g., administer 250 meg Misoprostol), notifications (e.g., notify OB of patient stage of blood loss protocol, notify blood bank of patient blood type), preparations (e.g., prepare transfusion equipment), or any suitable combination thereof. In some example embodiments, some or all elements are displayed in the order in which they should be completed. In some example embodiments, some or all elements are arranged based on multiple organizational methods (e.g., into categories, and then in order of expected or instructed completion within each category). However, elements generally may be arranged on the display in any suitable manner.

In some example embodiments, the providing of the guidance additionally or alternatively includes providing guidance through one or more audio cues, haptic cues, or any suitable combination thereof. For example, guidance (e.g., one or more selected elements from the current patient stage) may be recited or otherwise outputted from a speaker device. As another example, one or more haptic cues (e.g., vibration from a vibrational motor, with or without blinking lights, from a mobile computing device or other device) may be associated with one or more elements of guidance (e.g., color coded to a particular recommended action or category of actions). The providing of the guidance to the user may further include providing an indication when the guidance is updated. In some example embodiments, an audio or visual cue may indicate that updated guidance has been provided. This may provide the benefit of alerting the user that a change has been made to the displayed elements. As discussed above, the guidance may be updated in response to an input (e.g., a manual or automatic user input indicating the occurrence of an action or event), an updated patient blood loss stage (e.g., based on an updated estimated patient blood loss), or both. Thus, a notification may be provided when the guidance is updated based on (e.g., as a result of) user input, an updated patient stage, or both. Any suitable notification may be used to notify the user of the updated guidance, such as a sound (e.g., a ring, beep, ping, buzz, or any suitable combination thereof), vibration, flashing, a color change, a dynamic visual showing the elements being swapped out, or some or all of the blood loss protocol being scrolled through, any suitable combination thereof, or any other suitable cue. In other words, one or more alerts or notifications may be provided to prompt the user to view the guidance (e.g., output a buzz or beep to draw the user's attention to the display on which the guidance is provided).

The displaying of the guidance to the user may include displaying guidance comprised of one or more elements selected from the current patient stage. However, the displaying of the guidance may also include displaying one or more elements from additional patient stages. For example, the displaying of the guidance to the user may include displaying guidance from a previous patient stage, for example, if there were one or more elements that were not completed by the user in the previous patient stage and that should still be completed. Further, the providing of the guidance may include providing one or more notifications that the user has not performed an element. Such a notification may be presented visually, such as on a display screen of a computational device (e.g., flashing, blinking, changing the color of an item, or any suitable combination thereof), via one or more audio cues (e.g., by providing a noise or verbal cue to indicate that an element has not been completed), via one or more haptic cues (e.g., by causing vibration from a vibrational motor in a mobile device), or any suitable combination thereof. In some example embodiments, the providing and the displaying of the guidance may include presenting one or more elements from a subsequent patient stage. For example, if an estimated patient blood loss is increasing, and the patient is nearing the next severe patient stage, a processor may begin to "queue up" one or more additional elements from a subsequent patient stage in anticipation of the patient's worsening state. In some example embodiments, one or more elements from the subsequent stage may be presented along with one or more elements from the current patient stage. Elements from the subsequent patient stage may be distinguished from elements from the current patient stage. For example, elements from the subsequent stage may be a different color, a different intensity, or may be separated by a line or marker to differentiate elements from different stages. For example, FIG. 7 depicts an example GUI for a display showing elements from stages 2 and 3 of a blood loss protocol. The elements from patient stage 2 appear at the top of the screen, beneath a header labelled "STAGE 2," and the elements from patient stage 3 appear at the bottom of the screen beneath the "STAGE 3" header, and are separated from the stage 2 elements by a dashed line.

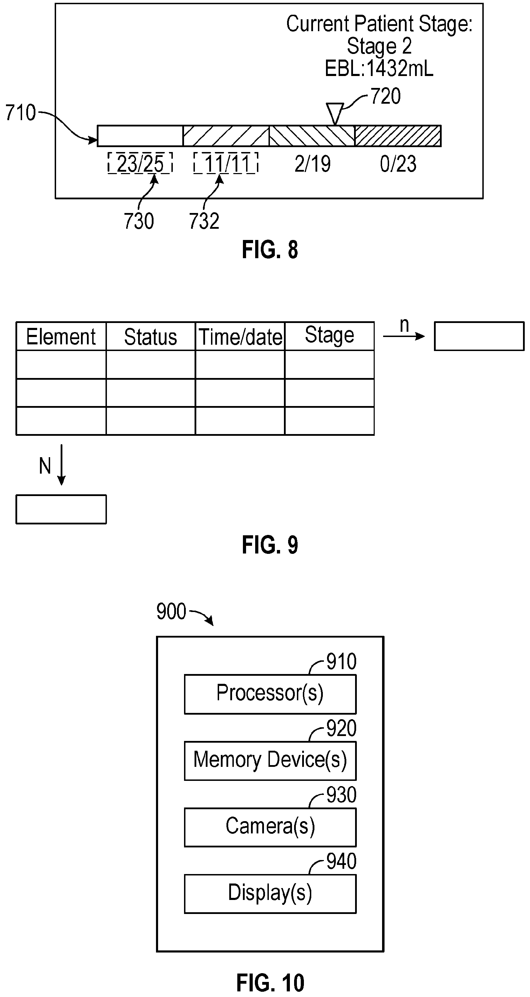

The providing of the guidance to the user may include displaying one or more summary graphics, such as a chart or a progress bar, of the patient's current blood loss in relation to the patient stages of the blood loss protocol. FIG. 8 shows an example of a summary graphic of patient blood loss. The summary graphic includes a status bar 710 with four patient stages of blood loss, indicated by variations in shading or coloring (e.g., greyscale intensity) from left to right. The current patient stage of blood loss in relation to the four patient stages is indicated by a marker icon 720, such as a triangle. The current patient stage, along with the estimated blood loss, is also indicated in the display. The summary graphic may show how many elements or steps in the blood loss protocol associated with each stage were completed by the user, in relation to how many total steps were presented to the user. That is, each section of the status bar (e.g., each associated with a different patient stage) may have a fraction 730, 732 near (e.g., below) it, indicating the number of completed steps in relation to the number of presented steps. For example, in the example summary graphic shown in FIG. 7, the first patient stage of blood loss is labeled with a fraction 730 indicating that 23 out of 25 steps were completed for this patient stage. As another example, the second patient stage of blood loss is labeled with a fraction 732 indicating that 11 out of 11 steps were completed for this patient stage. Various information (e.g., current patient stage, location of the marker icon, steps completed, etc.) may be updated throughout the medical procedure. FIG. 8 shows an example a summary graphic, but any suitable graphic, such as charts, graphs, flowcharts, tables, or any suitable combination thereof, may be used to summarize information related to patient blood loss and track progress in compliance with the blood loss protocol. Further, any type of information may be displayed, including current patient blood loss, current patient stage of a blood loss protocol, number of elements completed in each stage, number of elements not completed in each stage, length of time of a procedure, estimated length of time until the patient reaches the next stage, length of time the patient spent in each stage, patient data such as patient vitals (e.g., heart rate, oxygen levels, temperature, etc.), any other suitable information, or any suitable combination thereof. For example, FIG. 13 shows an example GUI display 1200 providing information about the current patient stage 1210, the estimated cumulative blood loss of the patient 1220, and a breakdown of the sources 1230 of the cumulative blood loss estimate (e.g., a prior estimated blood loss volume, blood loss from a container, such as a V-Drape, blood loss estimated from weighed items, etc.). The example GUI in FIG. 13 also provides an indication 1240 that a scale is ready to be used (e.g., to weigh soiled items to estimate additional patient blood loss), as well as two user input buttons 1250 and 1260. The input button 1250 allows the user to indicate an adjusted blood loss measurement from a fluid container (e.g., a "V-Drape"), and the input button 1260 allows the user to close the case, indicating that the procedure is complete or that the user is otherwise finished using the mobile application.

### Examples of Data Storage and Access

The providing of the guidance to the user may involve one or more of various methods of accessing and storing data. As described above, one or more blood loss protocols may be stored in the memory of one or more devices. To provide guidance to the user, one or more processors may access the one or more blood loss protocols stored in the memory. As described above, the providing of the guidance to the user may include selecting one or more elements from a blood loss protocol, and providing those one or more elements to the user. In some situations, it may be desirable to track and log information about the one or more elements that were selected from the blood loss protocol and displayed to the user. Thus, one or more of the methods discussed herein may include creating a data record of the one or more elements provided to the user during the medical procedure. Further, as previously described, the guiding of the user during the medical procedure may include receiving input indicative of whether a particular element was completed. For example, an element provided to the user may be a recommendation that the user administer an IV to the patient. When the user completes this element, a processor may receive an indication that the element has been completed. The indication may be an automatically transmitted indication (e.g., a wireless communication from the IV system indicating that it was properly filled and hooked up to the patient), or the indication may be in the form of a user input (e.g., the user operating a computational device to indicate completion of that particular element). In some example embodiments, a data record may store information regarding whether or more of the elements (e.g., as an item of guidance) provided to the user were completed or not. In some example embodiments, the determining of whether an element was not completed may include comparing the one or more elements provided to the user, with indications of the corresponding one or more actions being completed by the user. In some implementations, it may also be useful to track and store information about each element, such as: the time the element was completed or ignored; the time at which the element was provided, the medical procedure in which the element was provided; the hospital in which the medical procedure was being performed (e.g., conducted); the doctors, nurses, or other medical staff working on the medical procedure; the identity of the user who completed an item; any other relevant information; or any suitable combination thereof. Thus, the data record may include any suitable type of information about each element provided to a user in addition to whether the element was completed.

The storing and accessing of information about the guidance provided during the medical may include creating and maintaining a data record with information about the one or more elements provided to the user during a procedure. FIG. 9 is a schematic depiction of an example data record used to store information about a procedure. Each box in the data record contains information associated with an element in a procedure. Each row contains information about one element, and each column represents a different piece of information about that element. In FIG. 9, the first column of the example data record contains an element descriptor, which is an indication of what the element was (e.g., the action that was recommended to the user). In some example embodiments, the data record may store element descriptors according to what was displayed to the user (e.g., an element description could be "administer a blood transfusion"). In other example embodiments, the data record may store element descriptors according to an element number, an element code, or an abbreviation. For example, providing the guidance "administer a blood transfusion" may be stored as "TRNSF" in the data record, or any suitable abbreviation, including a numerical code. In some example embodiments, a lookup table may be associated with the data record to indicate the full text of each element code or abbreviation. The second column in the example data record shown in FIG. 9 is a status indicator of the element, which indicates whether or not the element was completed. The third column in the data record shown in FIG. 9 is a timestamp (e.g., showing time, date, or both) indicating when the element status was received (e.g., when the element was either completed or ignored). However, in other example embodiments, the timestamp may additionally or alternatively be associated with when the element was provided to the user (e.g., as guidance). The fourth column in the data record of FIG. 9 is the current patient stage of the blood loss protocol (e.g., the patient stage of the blood loss protocol that the patient's blood loss was associated with at the time the element was provided, or the time the element was completed or ignored). Although FIG. 9 includes four labeled columns, a data record may include any suitable number N of rows and any suitable number n of columns to store various amounts of information associated with the elements of the blood loss protocol. Non-limiting examples of such information that may be stored in a data record include: one or more element descriptors, one or more status indicators (e.g., completed or ignored), one or more timestamps (e.g., "time/date") of status, one or more timestamps of display to one or more users, an elapsed time of the medical procedure, the current patient stage of blood loss protocol, one or more patient parameters (e.g., height, weight, sex, BMI, medical history, etc.), procedure information, hospital information, the user who completed or ignored one or more elements, the estimated blood loss of the patient, an indication of the patient's risk of hemorrhage or other complications, an estimate of the time at which the patient will reach an unacceptable level of blood loss, any other suitable information, or any suitable combination thereof.

The data record generated during the medical procedure may be used for various purposes, such as during the medical procedure, after the medical procedure is complete, or both. During the medical procedure, the data record may be accessed by one or more processors to obtain information about what elements have already been provided to the user, as well as any other pertinent information about the provided elements (e.g., status, time, stage, etc.). For example, as described above, certain elements of the blood loss protocol may be conditionally selectable. Thus, the providing of guidance to the user may include accessing the data record during the medical procedure to determine whether a conditionally selectable element is currently selectable for provision to the user as guidance. The data record may be used to provide indications to the user regarding what elements have been completed. For example, when displaying one or more indications that indicate whether the user has completed an element, a processor may access the data record to determine, confirm, or both, whether the element has been completed to accordingly determine whether an indication that the element has been completed should be displayed. Furthermore, information from the data record may be used to transmit information during the medical procedure. For example, the providing of the guidance may include providing information to hospital administrative staff when an element provided to the user is ignored. Thus, the data record may be accessed to notify administrative staff of an ignored element. Information from the data record may also be used to determine use compliance with provided guidance, as discussed below.

It may be desirable to access the data record after the medical procedure is complete. At the end of the medical procedure, the data record represents a partial or full sequence of events of the medical procedure. Thus, the data record may be used in various ways to provide information about the medical procedure. For example, the data record may be stored and associated with an electronic medical record of the patient. As another example, the data record may be used by hospital administrative staff to determine compliance with the blood loss protocol from which one or more elements were provided to one or more users. This information may be used to determine if a particular user needs to improve compliance with prescribed protocols. The data record may be used to analyze the effect of certain actions on patient outcomes. For example, information from the data record may be used to refine content of the blood loss protocol (e.g., add an element, remove an element, rearrange elements, reword an element, change the nature of a conditionally selectable element or a conditionally unselectable element, or otherwise amend one or more elements of the blood loss protocol) in an effort to improve patient outcomes. Information from the data record can be communicated in any suitable manner. In some example embodiments, the data record may be exported, sent in an email, saved to a server, printed, uploaded or saved to a cloud-based storage system, or any suitable combination thereof. The data record may be stored in any suitable format. In some example embodiments, the storing of information from the data record includes formatting the information into a chart, spreadsheet, list, or any suitable combination thereof. In other example embodiments, the storing and communicating of such information may include generating charts or graphs based on the data in the data record. Further, the raw data in the data record may be exported and/or manipulated in any suitable manner, by any suitable program. Other Example Variations

One or more of the methods discussed herein may include providing guidance during the medical procedure based on one or more other suitable kinds of medical parameter protocols. For example, such methods may include estimating a medical parameter of the patient and using the estimated medical parameter to inform the guidance provided to the user. In some example embodiments, such as in the method depicted in FIG. 15, a method 1400 of providing guidance to a user may include estimating 1410 a medical parameter of a patient, determining 1420 a current patient stage of a medical parameter protocol based on the estimated medical parameter, and providing 1430 guidance to the user based at least in part on the current patient stage. Generally, the method 1400 may be similar to one or more of the methods described above with respect to providing guidance based on a blood loss protocol, except in the method 1400, the guidance may additionally or alternatively be associated with one or more other medical parameters. The providing of such guidance based on one or more medical parameter protocols may, for example, help ensure prompt and accurate adherence to one or more best practices, one or more other protocols for patient medical care (e.g., administration of medication, other actions), or any suitable combination thereof, thereby improving patient outcomes.

Examples of such medical parameters include: blood loss, blood pressure, hematocrit, hemoglobin concentration, concentration of another blood component, heart rate, blood coagulation (e.g., characterized by TEG), temperature, oxygen saturation, height, weight, age, BMI, sex, body fat percentage, intravenous fluid delivered, or any suitable combination thereof. One or more of these medical parameters may be estimated using one or more of the methods described herein (e.g., estimated blood loss or estimated blood component concentration), suitable instrumentation (e.g., sphygmomanometer, scale, pulse oximeter, thermometer, flow meter coupled to an TN setup, etc.), or any suitable combination thereof.

A medical parameter protocol may include a plurality of patient stages based on at least one estimated medical parameter, for example, and each patient stage may include one or more elements. In some example embodiments, one or more of the elements of a patient stage may include one or more recommended actions to be performed by the user to manage a health characteristic of the patient (e.g., restore a medical parameter to an accepted or normal range). In some example embodiments, the determining of the current patient stage may involve comparing the estimated medical parameter with one or more ranges of medical parameter values. Each range may be associated with a corresponding patient stage of the medical parameter protocol. Thus, an estimated medical parameter for the patient may be associated with a corresponding patient stage of the medical parameter protocol to determine the current patient stage, for example, by comparing the estimated medical parameter to medical parameter value ranges associated with one or more of various stages of the medical parameter protocol.

Furthermore, in some example embodiments, multiple estimated medical parameters are used individually or in suitable combination to determine the current patient stage in the medical parameter protocol. As an example, if a patient has a high heart rate (e.g., greater than 120 bpm) for a given age (e.g., greater than 70 years), as well as high systolic blood pressure (e.g., greater than 160mm Hg), then the method of providing guidance to the user may include determining a current patient stage for the patient based on the combination of these medical parameters. For example, the current patient stage may be determined by comparing each medical parameter to medical parameter value ranges associated with various patient stages of the medical parameter protocol.

The method may then include providing guidance (e.g., a series of protocol instructions specific to, or associated with, the current patient stage based on this combination of medical parameters), which may include, for example, administering certain amounts and quantities of drugs to manage the patient back to normal medical ranges. Guidance specific to the values or ranges of values for the medical parameter may be provided based on the protocol instructions for the associated conditions. The providing of such guidance may involve selecting one or more elements from the current patient stage of the medical parameter protocol. The providing of the guidance may further include displaying the guidance (e.g., comprised of the selected elements) to the user via a display screen (e.g. that of a mobile computing device), or providing the guidance in any suitable manner (e.g., other notifications such as other visual notifications, audio notifications, tactile notifications, etc.).

### Overview of Example Systems

The example systems described herein implement one or more of the example methods discussed herein for providing guidance to a user. Such systems for providing guidance to the user may include one or more processors configured to estimate the blood loss of the patient, determine the current patient stage of the blood loss protocol based on the estimated blood loss, and provide guidance to the user based on the current patient stage of the blood loss protocol. As described above, one or more processors of such a system for providing guidance to the user may be configured to select one or more elements from the current patient stage of a blood loss protocol. Further, the one or more processors may be further configured to provide updated guidance to the user based on user input, an updated patient stage of the blood loss protocol, or both. Such a system may include a display configured to display information to the user. Similarly, such a system for providing guidance to the user may include one or more processors configured to estimate a medical parameter of the patient, determine the current patient stage of a medical parameter protocol based on the estimated medical parameter, and provide guidance to the user based on the current patient stage of the medical parameter protocol.

As shown in FIG. 10, a system 900 for guiding a user during a medical procedure may include at least one processor 910 and a memory 920 for storing instructions to be carried out by the processor 910, data generated during the medical procedure, or both. In some example embodiments, the instructions to be carried out by the processor 910 may involve the use of one or more blood loss protocols or one or more other medical parameter protocols, also stored in the memory 920. For example, the system 900 may include one or more processors 910 configured to execute one or more aspects of the methods described herein as part of a mobile application executable on a mobile computing device. As described above, the blood loss protocol may include one or more patient stages, each associated with one or more ranges of blood loss values, and each patient stage may include one or more elements. Similarly, a medical parameter protocol may include one or more patient stages, each associated with one or more ranges of medical parameter values, and each patient stage may include one or more elements. Generally, the system 900 may be configured to substantially perform one or more of the methods described in further detail above. As further shown in FIG. 10, the system 900 may include a camera 930 for obtaining one or more images, and the system 900 may include a display 940 for displaying guidance or other information to the user. In some example embodiments, some or all of the system 900 may be in an integrated device and placed near the patient, the user, or both, during the medical procedure (e.g., in the operating room) to guide the user during the medical procedure. For example, the system 900 may at least partially include a handheld or mobile electronic computing device. Such a handheld or mobile device may, for example, be a tablet computer, laptop computer, mobile smartphone, or any suitable combination thereof, and such a device may include a camera (e.g., camera 930), a processor (e.g., processor 910), and a display (e.g., display 940). However, in other example embodiments, some or all of the system components may be separated as discrete, interconnected devices. For example, the camera 930, the display 940, or both, may be located substantially near the patient, the user, or both, during the medical procedure (e.g., in the operating room), while the processor 910 may be located at a remote location (e.g., in the operating room separate from the camera, the display, or both, or outside the operating room), communicating with the camera 930 and the display 940 through a wired or wireless connection or other network.

Generally, one or more processors 910 may be configured to execute the instructions that are stored in memory 920 such that, when the one or more processors 910 execute the instructions, the one or more processors 910 perform one or more aspects (e.g., operations) of the methods described herein. The instructions may be executed by computer-executable components integrated with the application, applet, host, server, network, website, communication service, communication interface, hardware/firmware/software elements of a user computer or mobile device, wristband, smartphone, or any suitable combination thereof. The instructions may be stored on memory or other computer-readable medium such as RAMs, ROMs, flash memory, EEPROMs, optical devices (e.g., CD or DVD), hard drives, floppy drives, or any suitable device.

As described above, the one or more processors 910 may be integrated into a handheld or mobile device. In other example embodiments, the one or more processors 910 may be incorporated into a computing device or system, such as a cloud-based computer system, a mainframe computer system, a grid-computer system, or other suitable computer system. Additionally or alternatively, the one or more processors 910 may be incorporated into a remote server that receives information about estimated patient blood loss, compares estimated patient blood loss to threshold values defining ranges associated with patient stages of a blood loss protocol to determine a current patient stage, and selects elements from the current patient stage to provide guidance to a user.

One or more of the example systems (e.g., system 900) discussed herein may include the camera 930 (e.g., optical sensor) that functions to generate one or more images, such as a set of one or more still images or as part of a video feed. One o more images may be used to estimate the blood loss from the patient, as described above. The camera 930 may include at least one optical image sensor (e.g., CCD, CMOS, etc.) that captures a color optical digital image with red, green, and blue (RGB) color components for the pixels, other suitable optical components, or any suitable combination thereof. For example, the camera 930 may include a single image sensor paired with one or more suitable corresponding optics, one or more filters (e.g., one or more color filter arrays such as a Bayer pattern filter), or any suitable combination thereof. As another example, the camera 930 may include multiple image sensors paired with suitable corresponding optics, such as at least one prism or diffractive surface to divide white light into separate color channels (e.g., RGB), each of which is detected by a respective image sensor. However, the camera 930 may include any suitable image sensors or other optics components to enable the camera 930 to generate images. The camera 930 may be configured to generate any number or type of suitable images, such as images depicting fluid-containing substrates, such as fluid containers (e.g., canisters), surgical items (e.g., surgical textiles), or any suitable combination thereof.

The camera 930 may be configured to transmit images to the processor 910 for analysis, to a database that stores the images, or to both. As previously described, the camera 930 may be integrated in the same device as one or more of the other components of the system 900, or the camera may be a separate component that communicates the image data to the other components.

One or more of the example systems (e.g., system 900) discussed herein may include the display 940 that functions to display or otherwise communicate guidance or other information to the user (e.g., a doctor, a nurse, etc.), and such information may be generated by the system 900, including one or more elements of a patient stage of a blood loss protocol (e.g., actions to be performed by a user), patient information, estimated blood loss of the patient, the current patient stage of the blood loss protocol, one or more notifications, a summary graphic of the blood loss protocol and the patient blood loss, one or more images of surgical textiles, one or more quantified metrics characterizing fluid in a surgical textile, or any suitable combination thereof. The display 940 may include a screen on a handheld or mobile device, a computer monitor, a television screen, a projector screen, or other suitable display.

In some example embodiments, the display 940 is configured to display a user interface (e.g., a GUI) that enables the user to interact with displayed information. For example, the user interface may enable the user to provide an indication that the user has completed an element of the blood loss protocol; provide information about the patient, procedure, hospital, etc.; manipulate images of fluid containing substrates, (e.g., zoom, crop, rotate, etc.); or any suitable combination thereof. As another example, the user interface may enable the user to select one or more display options (e.g., font, color, language, etc.), various content (e.g., patient information, quantified metrics or other fluid- related information, information about current patient stage of a blood loss protocol, alerts, etc.), or any suitable combination thereof. In such example embodiments, the display 940 may be user-interactive and include a resistive or capacitive touch screen that is responsive to skin, a stylet, or other user contact. In other example embodiments, the display 940 may be user-interactive via a cursor controlled by a mouse, keyboard, or other input device.

In some example embodiments, the system 900 may additionally or alternatively include an audio system that communicates information to the user. The display 940, the audio system, or both, may provide the current audio cues or other notifications to the user, which may assist the user in performing elements of a blood loss protocol or other medical parameter protocol.

As shown in FIG. 10, the display 940 may be integrated in the same device as one or more components of the system 900. Additionally or alternatively, the display 940 may include a standalone separate monitor, screen, or other suitable display.

In some example embodiments, one or more of the systems discussed herein (e.g., system 900) may also include items useful for tracking and quantifying patient blood loss, such as substrates configured to contain fluid, such as blood. For example, such systems may include fluid canisters, surgical items such as surgical textiles, or any suitable combination thereof. Such systems may also include one or more items configured to detect or otherwise determine the weight of an object, such as a scale. Further, such systems may include stands or mounts configured to hold mobile devices on which guidance is to be displayed.

Furthermore, such systems may include one or more items configured to estimate one or more other medical parameters, such as medical parameters for use in managing patient healthcare based on a medical parameter protocol. For example, one or more instruments to measure blood pressure, hematocrit, hemoglobin concentration, concentration of another blood component, heart rate, blood coagulation (e.g., characterized by TEG), temperature, oxygen saturation, height, weight, age, BMI, sex, body fat percentage, intravenous fluid delivered, etc., or any suitable combination thereof. Such instruments, such as a sphygmomanometer, a scale, a pulse oximeter, a thermometer, a flow meter coupled to an IV setup, etc. may be included.

The foregoing description, for purposes of explanation, used specific nomenclature to provide a thorough understanding of the present subject matter. However, it will be apparent to one skilled in the art that specific details are not required in order to practice the present subject matter. Thus, the foregoing descriptions of specific embodiments of the present subject matter are presented for purposes of illustration and description. They are not intended to be exhaustive or to limit the present subject matter to the precise forms disclosed. The invention is defined by the appended claims.

## Claims

1. A method (100; 500) comprising:
estimating (110; 510), by one or more processors (910) of a machine, an amount of blood lost by a patient during a procedure; and
determining (120; 520), by the one or more processors (910) of the machine, a current stage among a plurality of stages of a blood loss protocol (200), the current stage of the blood loss protocol (200) being determined based on the estimated amount of blood lost by the patient during the procedure;
**characterized in that** the method further comprises:
determining whether a trigger event occurred, the trigger event corresponding to a conditionally selectable element of a graphical presentation of guidance that corresponds to the determined current stage of the blood loss protocol (200); and
causing, by the one or more processors (910) of the machine, a display to present (130; 530) the graphical presentation of guidance that corresponds to the determined current stage among the plurality of stages of the blood loss protocol (200), the determining of whether the trigger event occurred further determining whether the corresponding conditionally selectable element is included in the guidance that corresponds to the determined current stage of the blood loss protocol (200).

2. The method (100; 500) of claim 1, wherein:
the current stage of the blood loss protocol (200) corresponds to a set of recommendable actions performable on the patient; and
the presentation of the guidance recommends performance of an action among the set of recommendable actions.

3. The method (100; 500) of claim 1, wherein:
the determining of the current stage of the blood loss protocol (200) is based on a comparison of the estimated amount of blood lost by the patient to a range (212, 222, 232, 242) of blood loss values, the range (212, 222, 232, 242) corresponding to the current stage of the blood loss protocol (200).

4. The method (100; 500) of claim 3, wherein:
multiple ranges of blood loss values correspond to the current stage of the blood loss protocol (200); and
the determining of the current stage of the blood loss protocol (200) includes selecting the range that corresponds to the current stage of the blood loss protocol (200) based on a medical parameter of the patient or a user input.

5. The method (100; 500) of claim 1, wherein:
the current stage of the blood loss protocol (200) corresponds to a set of recommendable actions performable on the patient; and
the presentation of the guidance indicates a recommended action selected from the set of recommendable actions performable on the patient.

6. The method (500) of claim 1, wherein:
the estimating (510) of the amount of blood lost by the patient includes updating (540) a previous estimate of the amount blood lost by the patient;
the determining (520) of the current stage of the blood loss protocol (200) includes updating (545) a previous determination of the current stage of the blood loss protocol (200) based on the updated estimate of the amount of blood lost by the patient; and
the presentation of the guidance (530) presents guidance (560) updated based on the updated determination of the current stage of the blood loss protocol (200).

7. A system (900) comprising:
a display (940);
one or more processors (910); and
a memory (920) storing instructions that, when executed by the one or more processors (910), cause the one or more processors (910) to perform operations comprising:
estimating (110; 510) an amount of blood lost by a patient during a procedure; and
determining (120; 520) a current stage among a plurality of stages of a blood loss protocol (200), the current stage of the blood loss protocol (200) being determined based on the estimated amount of blood lost by the patient during the procedure;
**characterized in that** the memory (920) further stores instructions that, when executed by the one or more processors (910), cause the one or more processors (910) to perform operations comprising:
determining whether a trigger event occurred, the trigger event corresponding to a conditionally selectable element of a graphical presentation of guidance that corresponds to the determined current stage of the blood loss protocol (200); and
causing the display (940) to present the graphical presentation of guidance (130; 530) that corresponds to the determined current stage among the plurality of stages of the blood loss protocol (200), the determining of whether the trigger event occurred further determining whether the corresponding conditionally selectable element is included in the guidance that corresponds to the determined current stage of the blood loss protocol (200).

8. The system (900) of claim 7, wherein:
the current stage of the blood loss protocol (200) corresponds to a set of recommendable actions performable on the patient; and
the presentation of the guidance recommends performance of an action among the set of recommendable actions.

9. The system (900) of claim 7, wherein:
the determining (120; 520) of the current stage of the blood loss protocol is based on a comparison of the estimated amount of blood lost by the patient to a range (212, 222, 232, 242) of blood loss values, the range (212, 222, 232, 242) corresponding to the current stage of the blood loss protocol (200).

10. The system (900) of claim 9, wherein:
multiple ranges of blood loss values correspond to the current stage of the blood loss protocol (200); and
the determining (120; 520) of the current stage of the blood loss protocol (200) includes selecting the range that corresponds to the current stage of the blood loss protocol (200) based on a medical parameter of the patient or a user input.

11. The system (900) of claim 7, wherein:
the current stage of the blood loss protocol (200) corresponds to a set of recommendable actions performable on the patient; and
the presentation of the guidance indicates a recommended action selected from the set of recommendable actions performable on the patient.

12. The system (900) of claim 7, wherein:
the estimating (510) of the amount of blood lost by the patient includes updating (540) a previous estimate of the amount blood lost by the patient;
the determining (520) of the current stage of the blood loss protocol (200) includes updating (545) a previous determination of the current stage of the blood loss protocol (200) based on the updated estimate of the amount of blood lost by the patient; and
the presentation of the guidance (530) presents guidance (560) updated based on the updated determination of the current stage of the blood loss protocol (200).

13. A machine-readable medium comprising instructions that, when executed by one or more processors (910) of a machine, cause the machine to perform operations comprising:
estimating (110; 510) an amount of blood lost by a patient during a procedure; and
determining (120; 520) a current stage among a plurality of stages of a blood loss protocol (200), the current stage of the blood loss protocol (200) being determined based on the estimated amount of blood lost by the patient during the procedure;
**characterized in that** the machine-readable medium comprise further instructions that, when executed by the one or more processors (910) of the machine, cause the machine to perform operations comprising:
determining whether a trigger event occurred, the trigger event corresponding to a conditionally selectable element of a graphical presentation of guidance that corresponds to the determined current stage of the blood loss protocol (200); and
causing a display to present (130; 530) the graphical presentation of guidance determined current stage among the plurality of stages of the blood loss protocol (200), the determining of whether the trigger event occurred further determining whether the corresponding conditionally selectable element is included in the guidance that corresponds to the determined current stage of the blood loss protocol (200).

14. The machine-readable medium of claim 13, wherein:
the current stage of the blood loss protocol (200) corresponds to a set of recommendable actions performable on the patient; and
the presentation of the guidance recommends performance of an action among the set of recommendable actions.

15. The machine-readable medium of claim 13, wherein:
the determining (120; 520) of the current stage of the blood loss protocol (200) is based on a comparison of the estimated amount of blood lost by the patient to a range (212, 222, 232, 242) of blood loss values, the range (212, 222, 232, 242) corresponding to the current stage of the blood loss protocol (200).

## Patentansprüche

1. Verfahren (100; 500), umfassend:
Schätzen (110; 510) einer Menge an von einem Patienten während eines Eingriffs verlorenem Blut durch einen oder mehrere Prozessoren (910) einer Maschine; und
Bestimmen (120; 520) einer aktuellen Stufe aus einer Mehrzahl von Stufen eines Blutverlustprotokolls (200) durch den einen oder die mehreren Prozessoren (910) der Maschine, wobei die aktuelle Stufe des Blutverlustprotokolls (200) basierend auf der geschätzten Menge an vom Patienten während des Eingriffs verlorenem Blut bestimmt wird;
**dadurch gekennzeichnet, dass** das Verfahren ferner umfasst:
Bestimmen, ob ein Auslöseereignis eingetreten ist, wobei das Auslöseereignis einem bedingt auswählbaren Element einer grafischen Darstellung einer Anleitung entspricht, die der bestimmten aktuellen Stufe des Blutverlustprotokolls (200) entspricht; und
Veranlassen durch den einen oder die mehreren Prozessoren (910) der Maschine, dass eine Anzeige (130; 530) die grafische Darstellung der Anleitung darstellt, die der bestimmten aktuellen Stufe aus der Mehrzahl von Stufen des Blutverlustprotokolls (200) entspricht, wobei das Bestimmen, ob das Auslöseereignis eingetreten ist, ferner ein Bestimmen umfasst, ob das entsprechende bedingt auswählbare Element in der Anleitung enthalten ist, die der bestimmten aktuellen Stufe des Blutverlustprotokolls (200) entspricht.

2. Verfahren (100; 500) nach Anspruch 1, wobei:
die aktuelle Stufe des Blutverlustprotokolls (200) einer Reihe empfehlenswerter Maßnahmen entspricht, die am Patienten durchführbar sind; und
die Darstellung der Anleitung eine Durchführung einer Maßnahme aus der Reihe empfehlenswerter Maßnahmen empfiehlt.

3. Verfahren (100; 500) nach Anspruch 1, wobei:
das Bestimmen der aktuellen Stufe des Blutverlustprotokolls (200) auf einem Vergleich der geschätzten Menge an vom Patienten verlorenem Blut mit einem Bereich (212, 222, 232, 242) von Blutverlustwerten basiert, wobei der Bereich (212, 222, 232, 242) der aktuellen Stufe des Blutverlustprotokolls (200) entspricht.

4. Verfahren (100; 500) nach Anspruch 3, wobei:
mehrere Bereiche von Blutverlustwerten der aktuellen Stufe des Blutverlustprotokolls (200) entsprechen; und
das Bestimmen der aktuellen Stufe des Blutverlustprotokolls (200) ein Auswählen des Bereichs umfasst, der der aktuellen Stufe des Blutverlustprotokolls (200) entspricht, basierend auf einem medizinischen Parameter des Patienten oder einer Benutzereingabe.

5. Verfahren (100; 500) nach Anspruch 1, wobei:
die aktuelle Stufe des Blutverlustprotokolls (200) einer Reihe empfehlenswerter Maßnahmen entspricht, die am Patienten durchführbar sind; und
die Darstellung der Anleitung eine empfohlene Maßnahme angibt, die aus der Reihe der am Patienten durchführbaren empfehlenswerten Maßnahmen ausgewählt wurde.

6. Verfahren (500) nach Anspruch 1, wobei:
das Schätzen (510) der Menge an vom Patienten verlorenem Blut ein Aktualisieren (540) einer vorherigen Schätzung der Menge an vom Patienten verlorenem Blut umfasst;
das Bestimmen (520) der aktuellen Stufe des Blutverlustprotokolls (200) ein Aktualisieren (545) einer vorherigen Bestimmung der aktuellen Stufe des Blutverlustprotokolls (200) basierend auf der aktualisierten Schätzung der Menge an vom Patienten verlorenem Blut umfasst; und
die Darstellung der Anleitung (530) eine basierend auf der aktualisierten Bestimmung der aktuellen Stufe des Blutverlustprotokolls (200) aktualisierte Anleitung (560) darstellt.

7. System (900), umfassend:
eine Anzeige (940);
einen oder mehrere Prozessoren (910); und
einen Speicher (920), der Anweisungen speichert, die, wenn sie von dem einen oder den mehreren Prozessoren (910) ausgeführt werden, den einen oder die mehreren Prozessoren (910) veranlassen, Vorgänge durchzuführen, die Folgendes umfassen:
Schätzen (110; 510) einer Menge an von einem Patienten während eines Eingriffs verlorenem Blut; und
Bestimmen (120; 520) einer aktuellen Stufe aus einer Mehrzahl von Stufen eines Blutverlustprotokolls (200), wobei die aktuelle Stufe des Blutverlustprotokolls (200) basierend auf der geschätzten Menge an vom Patienten während des Eingriffs verlorenem Blut bestimmt wird;
**dadurch gekennzeichnet, dass** der Speicher (920) ferner Anweisungen speichert, die, wenn sie von dem einen oder den mehreren Prozessoren (910) ausgeführt werden, den einen oder die mehreren Prozessoren (910) veranlassen, Vorgänge durchzuführen, die Folgendes umfassen:
Bestimmen, ob ein Auslöseereignis eingetreten ist, wobei das Auslöseereignis einem bedingt auswählbaren Element einer grafischen Darstellung einer Anleitung entspricht, die der bestimmten aktuellen Stufe des Blutverlustprotokolls (200) entspricht; und
Veranlassen, dass die Anzeige (940) die grafische Darstellung der Anleitung (130; 530) darstellt, die der bestimmten aktuellen Stufe aus der Mehrzahl von Stufen des Blutverlustprotokolls (200) entspricht, wobei das Bestimmen, ob das Auslöseereignis eingetreten ist, ferner ein Bestimmen umfasst, ob das entsprechende bedingt auswählbare Element in der Anleitung enthalten ist, die der bestimmten aktuellen Stufe des Blutverlustprotokolls (200) entspricht.

8. System (900) nach Anspruch 7, wobei:
die aktuelle Stufe des Blutverlustprotokolls (200) einer Reihe empfehlenswerter Maßnahmen entspricht, die am Patienten durchführbar sind; und
die Darstellung der Anleitung die Durchführung einer Maßnahme aus der Reihe empfehlenswerter Maßnahmen empfiehlt.

9. System (900) nach Anspruch 7, wobei:
das Bestimmen (120; 520) der aktuellen Stufe des Blutverlustprotokolls auf einem Vergleich der geschätzten Menge an vom Patienten verlorenem Blut mit einem Bereich (212, 222, 232, 242) von Blutverlustwerten basiert, wobei der Bereich (212, 222, 232, 242) der aktuellen Stufe des Blutverlustprotokolls (200) entspricht.

10. System (900) nach Anspruch 9, wobei:
mehrere Bereiche von Blutverlustwerten der aktuellen Stufe des Blutverlustprotokolls (200) entsprechen; und
das Bestimmen (120; 520) der aktuellen Stufe des Blutverlustprotokolls (200) ein Auswählen des Bereichs umfasst, der der aktuellen Stufe des Blutverlustprotokolls (200) entspricht, basierend auf einem medizinischen Parameter des Patienten oder einer Benutzereingabe.

11. System (900) nach Anspruch 7, wobei:
die aktuelle Stufe des Blutverlustprotokolls (200) einer Reihe empfehlenswerter Maßnahmen entspricht, die am Patienten durchführbar sind; und
die Darstellung der Anleitung eine empfohlene Maßnahme angibt, die aus der Reihe der am Patienten durchführbaren empfehlenswerten Maßnahmen ausgewählt wurde.

12. System (900) nach Anspruch 7, wobei:
das Schätzen (510) der Menge an vom Patienten verlorenem Blut ein Aktualisieren (540) einer vorherigen Schätzung der Menge an vom Patienten verlorenem Blut umfasst;
das Bestimmen (520) der aktuellen Stufe des Blutverlustprotokolls (200) ein Aktualisieren (545) einer vorherigen Bestimmung der aktuellen Stufe des Blutverlustprotokolls (200) basierend auf der aktualisierten Schätzung der Menge an vom Patienten verlorenem Blut umfasst; und
die Darstellung der Anleitung (530) eine basierend auf der aktualisierten Bestimmung der aktuellen Stufe des Blutverlustprotokolls (200) aktualisierte Anleitung (560) darstellt.

13. Maschinenlesbares Medium, das Anweisungen umfasst, die, wenn sie von einem oder mehreren Prozessoren (910) einer Maschine ausgeführt werden, die Maschine veranlassen, Vorgänge durchzuführen, die Folgendes umfassen:
Schätzen (110; 510) einer Menge an von einem Patienten während eines Eingriffs verlorenem Blut; und
Bestimmen (120; 520) einer aktuellen Stufe aus einer Mehrzahl von Stufen eines Blutverlustprotokolls (200), wobei die aktuelle Stufe des Blutverlustprotokolls (200) basierend auf der geschätzten Menge an vom Patienten während des Eingriffs verlorenem Blut bestimmt wird;
**dadurch gekennzeichnet, dass** das maschinenlesbare Medium weitere Anweisungen umfasst, die, wenn sie von dem einen oder den mehreren Prozessoren (910) der Maschine ausgeführt werden, die Maschine veranlassen, Vorgänge durchzuführen, die Folgendes umfassen:
Bestimmen, ob ein Auslöseereignis eingetreten ist, wobei das Auslöseereignis einem bedingt auswählbaren Element einer grafischen Darstellung einer Anleitung entspricht, die der bestimmten aktuellen Stufe des Blutverlustprotokolls (200) entspricht; und
Veranlassen, dass eine Anzeige (130; 530) die grafische Darstellung der Anleitung darstellt, die der bestimmten aktuellen Stufe aus der Mehrzahl von Stufen des Blutverlustprotokolls (200) entspricht, wobei das Bestimmen, ob das Auslöseereignis eingetreten ist, ferner ein Bestimmen umfasst, ob das entsprechende bedingt auswählbare Element in der Anleitung enthalten ist, die der bestimmten aktuellen Stufe des Blutverlustprotokolls (200) entspricht.

14. Maschinenlesbares Medium nach Anspruch 13, wobei:
die aktuelle Stufe des Blutverlustprotokolls (200) einer Reihe empfehlenswerter Maßnahmen entspricht, die am Patienten durchführbar sind; und
die Darstellung der Anleitung eine Durchführung einer Maßnahme aus der Reihe empfehlenswerter Maßnahmen empfiehlt.

15. Maschinenlesbares Medium nach Anspruch 13, wobei:
das Bestimmen (120; 520) der aktuellen Stufe des Blutverlustprotokolls (200) auf einem Vergleich der geschätzten Menge an vom Patienten verlorenem Blut mit einem Bereich (212, 222, 232, 242) von Blutverlustwerten basiert, wobei der Bereich (212, 222, 232, 242) der aktuellen Stufe des Blutverlustprotokolls (200) entspricht.

## Revendications

1. Procédé (100; 500) comprenant:
l'estimation (110; 510), à l'aide d'un ou plusieurs processeurs (910) d'une machine, de la quantité de sang perdue par un patient au cours d'une intervention; et
la détermination (120 ; 520), à l'aide du ou des processeurs (910) de la machine, d'une étape actuelle parmi une pluralité d'étapes d'un protocole de perte sanguine (200), l'étape actuelle du protocole de perte sanguine (200) étant déterminée sur la base de la quantité estimée de sang perdu par le patient pendant l'intervention;
**caractérisé en ce que** le procédé comprend en outre:
la détermination si un événement déclencheur s'est produit, l'événement déclencheur correspondant à un élément sélectionnable sous condition d'une présentation graphique de recommandation qui correspond à l'étape actuelle déterminée du protocole de perte sanguine (200) ; et
le fait d'amener, par le ou les processeurs (910) de la machine, un écran à afficher (130; 530) la présentation graphique de recommandation qui correspond à l'étape actuelle déterminée parmi la pluralité d'étapes du protocole de perte sanguine (200), la détermination de la survenue de l'événement déclencheur déterminant en outre si l'élément sélectionnable sous condition correspondant est inclus dans la recommandation qui correspond à l'étape actuelle déterminée du protocole de perte sanguine (200).

2. Procédé (100; 500) selon la revendication 1, dans lequel:
l'étape actuelle du protocole de perte sanguine (200) correspond à un ensemble d'actions recommandées pouvant être effectuées sur le patient; et
la présentation de la recommandation préconise la mise en œuvre d'une action parmi l'ensemble des actions recommandées.

3. Procédé (100; 500) selon la revendication 1, dans lequel:
la détermination de l'étape actuelle du protocole de perte sanguine (200) est basée sur une comparaison entre la quantité estimée de sang perdu par le patient et une plage (212, 222, 232, 242) de valeurs de perte sanguine, la plage (212, 222, 232, 242) correspondant à l'étape actuelle du protocole de perte sanguine (200).

4. Procédé (100; 500) selon la revendication 3, dans lequel:
plusieurs plages de valeurs de perte sanguine correspondent à l'étape actuelle du protocole de perte sanguine (200); et
la détermination de l'étape actuelle du protocole de perte sanguine (200) consiste à sélectionner la plage qui correspond à l'étape actuelle du protocole de perte sanguine (200) sur la base d'un paramètre médical du patient ou d'une entrée de l'utilisateur.

5. Procédé (100; 500) selon la revendication 1, dans lequel:
l'étape actuelle du protocole de perte sanguine (200) correspond à un ensemble d'actions recommandées pouvant être effectuées sur le patient; et
la présentation de la recommandation indique une action recommandée sélectionnée parmi l'ensemble des actions recommandées pouvant être réalisées sur le patient.

6. Procédé (500) selon la revendication 1, dans lequel:
l'estimation (510) de la quantité de sang perdue par le patient consiste à mettre à jour (540) une estimation précédente de la quantité de sang perdue par le patient;
la détermination (520) de l'étape actuelle du protocole de perte sanguine (200) consiste à mettre à jour (545) une détermination précédente de l'étape actuelle du protocole de perte sanguine (200) sur la base de l'estimation mise à jour de la quantité de sang perdue par le patient; et
la présentation de la recommandation (530) présente la recommandation (560) mise à jour sur la base de la détermination mise à jour de l'étape actuelle du protocole de perte sanguine (200).

7. Système (900) comprenant:
un écran (940);
un ou plusieurs processeurs (910); et
une mémoire (920) stockant des instructions qui, lorsqu'elles sont exécutées par le ou les processeurs (910), amènent le ou les processeurs (910) à effectuer des opérations consistant à:
estimer (110; 510) une quantité de sang perdue par un patient au cours d'une intervention; et
déterminer (120; 520) une étape actuelle parmi une pluralité d'étapes d'un protocole de perte sanguine (200), l'étape actuelle du protocole de perte sanguine (200) étant déterminée sur la base de la quantité estimée de sang perdu par le patient pendant l'intervention;
**caractérisé en ce que** la mémoire (920) stocke en outre des instructions qui, lorsqu'elles sont exécutées par le ou les processeurs (910), amènent le ou les processeurs (910) à effectuer des opérations consistant à:
déterminer si un événement déclencheur s'est produit, l'événement déclencheur correspondant à un élément sélectionnable sous condition d'une présentation graphique de recommandation qui correspond à l'étape actuelle déterminée du protocole de perte sanguine (200); et
amener l'écran (940) à afficher la présentation graphique de la recommandation (130; 530) qui correspond à l'étape actuelle déterminée parmi la pluralité d'étapes du protocole de perte sanguine (200), la détermination de la survenue de l'événement déclencheur déterminant en outre si l'élément sélectionnable sous condition correspondant est inclus dans la recommandation qui correspond à l'étape actuelle déterminée du protocole de perte sanguine (200).

8. Système (900) selon la revendication 7, dans lequel:
l'étape actuelle du protocole de perte sanguine (200) correspond à un ensemble d'actions recommandées pouvant être effectuées sur le patient; et
la présentation de la recommandation préconise la mise en œuvre d'une action parmi l'ensemble des actions recommandées.

9. Système (900) selon la revendication 7, dans lequel:
la détermination (120; 520) de l'étape actuelle du protocole de perte sanguine est basée sur une comparaison entre la quantité estimée de sang perdu par le patient et une plage (212, 222, 232, 242) de valeurs de perte sanguine, la plage (212, 222, 232, 242) correspondant à l'étape actuelle du protocole de perte sanguine (200).

10. Système (900) selon la revendication 9, dans lequel:
plusieurs plages de valeurs de perte sanguine correspondent à l'étape actuelle du protocole de perte sanguine (200); et
la détermination (120; 520) de l'étape actuelle du protocole de perte sanguine (200) consiste à sélectionner la plage qui correspond à l'étape actuelle du protocole de perte sanguine (200) sur la base d'un paramètre médical du patient ou d'une entrée de l'utilisateur.

11. Système (900) selon la revendication 7, dans lequel:
l'étape actuelle du protocole de perte sanguine (200) correspond à un ensemble d'actions recommandées pouvant être effectuées sur le patient; et
la présentation de la recommandation indique une action recommandée sélectionnée parmi l'ensemble des actions recommandées pouvant être réalisées sur le patient.

12. Système (900) selon la revendication 7, dans lequel:
l'estimation (510) de la quantité de sang perdue par le patient consiste à mettre à jour (540) une estimation précédente de la quantité de sang perdue par le patient;
la détermination (520) de l'étape actuelle du protocole de perte sanguine (200) consiste à mettre à jour (545) une détermination précédente de l'étape actuelle du protocole de perte sanguine (200) sur la base de l'estimation mise à jour de la quantité de sang perdue par le patient; et
la présentation de la recommandation (530) présente la recommandation (560) mise à jour sur la base de la détermination mise à jour de l'étape actuelle du protocole de perte sanguine (200).

13. Support lisible par machine comprenant des instructions qui, lorsqu'elles sont exécutées par un ou plusieurs processeurs (910) d'une machine, amènent la machine à réaliser les opération consistant à:
estimer (110; 510) une quantité de sang perdue par un patient au cours d'une intervention; et
déterminer (120; 520) une étape actuelle parmi une pluralité d'étapes d'un protocole de perte sanguine (200), l'étape actuelle du protocole de perte sanguine (200) étant déterminée sur la base de la quantité estimée de sang perdu par le patient pendant l'intervention;
**caractérisé en ce que** le support lisible par machine comprenant des instructions qui, lorsqu'elles sont exécutées par un ou plusieurs processeurs (910) de la machine, amènent la machine à réaliser les opération consistant à:
déterminer si un événement déclencheur s'est produit, l'événement déclencheur correspondant à un élément sélectionnable sous condition d'une présentation graphique de recommandation qui correspond à l'étape actuelle déterminée du protocole de perte sanguine (200); et
amener un écran à afficher (130; 530) la présentation graphique de la recommandation déterminée parmi la pluralité d'étapes du protocole de perte sanguine (200), la détermination de la survenue de l'événement déclencheur déterminant en outre si l'élément sélectionnable sous condition correspondant est inclus dans la recommandation qui correspond à l'étape actuelle déterminée du protocole de perte sanguine (200).

14. Support lisible par machine selon la revendication 13, dans lequel:
l'étape actuelle du protocole de perte sanguine (200) correspond à un ensemble d'actions recommandées pouvant être effectuées sur le patient; et
la présentation de la recommandation préconise la mise en œuvre d'une action parmi l'ensemble des actions recommandées.

15. Support lisible par machine selon la revendication 13, dans lequel:
la détermination (120; 520) de l'étape actuelle du protocole de perte sanguine (200) est basée sur une comparaison entre la quantité estimée de sang perdu par le patient et une plage (212, 222, 232, 242) de valeurs de perte sanguine, la plage (212, 222, 232, 242) correspondant à l'étape actuelle du protocole de perte sanguine (200).
